# EUROPEAN PATENT APPLICATION

(11) **EP 3 406 714 A1**
(43) Date of publication of application: **28.11.2018**
(21) Application number: 16892410.8
(22) Date of filing: 03.03.2016
(51) Int. Cl.: C12N 5/09, C12N 5/00, C12N 5/095, G01N 33/574

(54) **METHOD FOR DETECTING OR SEPARATING/OBTAINING CIRCULATING TUMOR CELL EMPLOYING CELL PROLIFERATION METHOD**

(71) Applicant: Umezu, Yasuiki, Sendai-shi, Miyagi 981-0952 (JP)
(72) Inventor: Umezu, Yasuiki, Sendai-shi, Miyagi 981-0952 (JP)
(74) Representative: Carpmaels & Ransford LLP
(86) International application number: PCT/JP2016/001172
(87) International publication number: WO 2017/149564

(57) **Abstract**

An object is to provide a method for detecting or separating/obtaining CTC, which is capable of reliably and stably detecting or separating/obtaining a circulating tumor cell and a circulating tumor stem cell present in trace amounts in a biological circulating fluid such as blood or lymph, even in the state where the cancer type of the tumor cells cannot be determined yet and the state where the tumor cells are present in trace amounts in the biological circulating fluid. The object is attained by a method for detecting or separating/obtaining CTC and/or CTSC in a biological circulating fluid, comprising the following treatment steps (1) to (4): (1) a first step of pretreating a sample from the biological circulating fluid to obtain a mononuclear cell phase; (2) a second step of providing a well plate in which a culture medium consisting of a serum-free cell growth medium for circulating tumor cell and/or circulating tumor stem cell has been injected, and seeding thereto the mononuclear cells obtained in the first step, followed by incubation; (3) a third step of removing the culture medium from a well of the plate obtained by the incubation in the second step; and (4) a fourth step of detecting or separating/obtaining an adherent tumor cell attached to the well of the plate after the third step.

## Description

### Technical Field

The present invention relates to a method for detecting or separating/obtaining a circulating tumor cell (CTC) and a circulating tumor stem cell (CTSC) which are tumor (cancer) cells that invade a human biological tissue, i.e., a circulating body fluid typified by peripheral blood, or a circulating body fluid from various organs in the body, including the bone marrow, the spleen, and the like, via this body fluid, and particularly relates to providing a method for detecting or separating/obtaining a circulating tumor cell (CTC) and a circulating tumor stem cell (CTSC), which is capable of reliably and stably detecting or separating/obtaining a circulating tumor cell and a circulating tumor stem cell present in trace amounts in a biological circulating body fluid such as blood or lymph, even in the state where the cancer type of the tumor cells cannot be determined yet and the state where the tumor cells are present in trace amounts in the biological circulating body fluid (in the description below, the circulating tumor cell (CTC) and the circulating tumor stem cell (CTSC) are collectively referred to as a "circulating tumor cell (CTC)" except for the case where they need to be described distinctively, and the term "circulating tumor cell (CTC)" is meant to also include the "circulating tumor stem cell (CTSC)").

### Background Art

Cancer diseases rank high in death rates from illnesses in developed countries. Particularly, in Japan, although a decreasing trend is seen in statistics such as age-adjusted incidence rates and the death rates, the contribution of the aging of the population or chronic diseases such as diabetic mellitus to cancer development or deaths is high. Now, one out of two persons is stochastically affected by cancer, and one out of three persons find themselves in an exceedingly alarming situation in such a way that they die from cancer (Estimated using the method by Wum LM et al., Estimating lifetime and age-conditional probabilities of developing cancer, Lifetime Data Anal., 1998, 4: 169-186).

Meanwhile, to cope with these cancer diseases, headway is being made in various cancer treatment methods by focusing on the development of treatment techniques thereof. The advance of surgical therapy, chemotherapy, and radiotherapy as 3 major therapies of cancer treatment, and immunotherapy, etc. which has received attention in recent years has obtained reasonably outstanding results. However, postoperative recurrence, regional lymph node metastasis, distal metastasis, etc. cannot be sufficiently controlled yet, and it has become urgent to take measures thereagainst. Also, the case where postoperative recurrence and metastasis occur even if cancer tissues are successfully resected has been found in recent years, and it has also become known that the presence of cancer stem cells is very important as a factor of the postoperative recurrence and metastasis (Breast Cancer Res Treat 2010, 124: 403-412; New Engl. J Med 2004, 351: 781-791; Clin Cancer Res 2008, 14: 6302-6309; and J. Clin Oncol 2008, 26: 3213-3221). Furthermore, it has become evident that CTCs circulating in the body are involved in this recurrence and metastasis at the center of this mechanism (Clin Cancer Res 2008, 14: 7004-7010; and Proc (Bayl Univ Med Cent) 2008, 21: 127-132).

Cancer cells or tissues (hereinafter, which refer to general malignant tumors including leukemia) that have developed in human in vivo tissues or moreover various organs in the body form tumor tissues at a primary lesion (location of cancer development), except for leukemia, by binding proliferated cancer cells through adherent factors. However, the cancer cells are liberated from the primary lesion, without remaining at the primary lesion, due to the abnormality or disappearance of the adherent factors, degrade connective tissues, vascular vessels, and lymph vessel walls surrounding the cancer cells, and infiltrate into blood or lymph. The cancer cells that have infiltrated into the body fluid circulate as circulating tumor cells (CTCs) in the body, together with the blood or the lymph, which circulates in the body. The CTCs are carried to other tissues or organs by the circulation of the blood or the like, squeeze out from the vascular vessels or the like, in these tissues or organs, newly synthesize adherent factors, and colonize to form a metastatic lesion. The circulation of these CTCs in the body and the formation of a metastatic lesion are considered as a cancer metastasis mechanism and considered to be involved in cancer recurrence and metastasis.

In the establishment of the diagnosis or treatment of cancer, the early obtainment of information on the determination or pathological condition of cancer that has developed at a primary lesion, before the metastasis of the cancer to other tissues or organs occurs, is a very important factor for effectively performing the diagnosis or treatment of the cancer and offers useful information for preventing the metastasis of the cancer to other tissues or organs. The analysis of CTCs as means therefor has received attention as a very important factor. Also, the analysis of CTCs is considered to be also important for the prediction of cancer recurrence or the evaluation of the effect of cancer treatment. It has been reported that this means is also effective for the prediction of prognosis or the assessment of the effect of treatment.

However, the level of presence of CTCs circulating in blood or the like is very low, and the half-life of cancer cells circulating in blood is a short time such as 1 to 24 hours. Therefore, the detection of CTCs present in a circulating body fluid is in a very difficult situation. For example, the separation of CTCs from peripheral blood has poor efficiency. As for CTCs, the report of 19 facilities of joint research worldwide in 2012 (J Translational-medicine 2012, 10: 138: 1-20) has also reported that 1/10⁸ from mononuclear cells in blood, i.e., only one cell from 100 ml of blood, can be isolated. This is a very low probability of 50 cells in terms of a human adult having a body weight of 60 kg even if the whole amount of approximately 5 l (5000 ml) in total of peripheral blood is collected, and furthermore, only a very small number of CTCs can be isolated. This fact has been most disincentive to CTC research (J Translational-medicine 2012, 10: 138: 1-20).

In recent years, headway has been made in CTC detection or measurement techniques, and detection sensitivity or measurement accuracy has been improved by the advance of the detection or measurement techniques. It has become possible to specifically detect only several cancer cells present in 100,000 to 100,000,000 mononucleated cells of blood. Furthermore, this has become recognized as examination that produces clinical information, such as the prediction of prognosis or the assessment of the effect of treatment, on metastatic cancer such as breast cancer, colorectal cancer, or prostate cancer. Moreover, FDA (Food and Drug Administration) of the United States has recognized the clinical usefulness of CTCs for breast cancer, colorectal cancer, and prostate cancer, reaching the approval as an *in vitro* diagnostic. However, the current CTC detection or measurement techniques have a compelling reason to become detection or measurement techniques restricted to particular CTC, such as the case where intended cancer cells have already been determined. A CTC detection or measurement technique that can be applied to a wide range of cancer cells has not yet been found.

An immunomagnetic separation/detection method is known as a currently widely utilized method for detecting CTCs (Proc. Natl. Acad. Sci, USA, 95: 4589-4594, 1998; WO99/41613; and Japanese unexamined Patent Application Publication (Translation of PCT Application) No. 2002-503814). In this method, magnetic beads immobilized a monoclonal antibody against an epithelial cell adhesion molecule (EpCAM, "CD326") of epithelial cancer cells are used for binding the epithelial cell adhesion molecule (EpCAM) of CTCs as a target in a sample, and enriching the magnetically labeled CTCs by magnetic condensation, consequently detecting CTCs contained in trace amounts in the sample. Various methods for detecting CTCs are also disclosed by applying this immunomagnetic separation/detection method.

For example, in WO2007/133465 (Japanese unexamined Patent Application Publication (Translation of PCT Application) No. 2009-537021), disclosed are a method for rapidly and accurately detecting scarce CTCs in blood by labeling body fluid-derived immunomagnetic target CTCs with a fluorophore, and acquiring the labeled cells by scanning an image of two-dimensionally distributed CTCs by a time-delayed integrating imaging (TDI) technique with a beam homogenizer introduced therein, and an apparatus therefor. Also, in Japanese unexamined Patent Application Publication Nos. 2007-178193 and 2012-103077, disclosed is a method for detecting a gene in fluorescently stained cells at the same time with the counting of the number of the fluorescently stained cells by fluorescently staining cancer cells captured on magnetic beads immobilized with an epithelial cell surface antigen(EpCAM)-specific antibody, with a fluorescently labeled antibody against an epithelial specific surface antigen (cytokeratin) different from the surface antigen, and counting the number of the fluorescently labeled cells in combination with the staining of cell nuclei employing a fluorescently labeled DNA probe that hybridizes to an oncogene according to fluorescent *in situ* hybridization (FISH).

Furthermore, in Japanese unexamined Patent Application Publication No. 2014-105159, disclosed is a method for detecting or quantitatively analyzing CTCs with the cell viability maintained by the condensation of CTCs using magnetic beads immobilized a monoclonal antibody (first antibody) against a human-derived epithelial cell adhesion molecule (EpCAM), a fluorescently labeled anti-EpCAM monoclonal antibody (second antibody) that specifically recognizes a different epitope, and a method for staining cell nuclei (intact CTC enumeration and analysis procedure: iCeap). In Japanese unexamined Patent Application Publication No. 2014-112094, disclosed is a method for identifying and characterizing CTCs employing a cytokeratin (CK) marker which is a marker specific for epithelial cells, and a second marker which is a cytological dye that identifies CTCs by morphology, dimension or a nucleus-to-cytoplasm ratio, such as a Wright-Giemsa dye.

Furthermore, in Japanese unexamined Patent Application Publication No. 2014-39480, for the detection of EpCAM negative CTCs, disclosed is a method for detecting a urokinase-specific substrate-derived signal and separating and recovering CTCs by providing a culture surface containing a urokinase (urokinase-type plasminogen activator: uPA)-specific fluorescent substrate, and seeding a blood sample on the culture surface, followed by incubation. As mentioned above, various detection methods of detecting CTCs employing a cell marker (specific antigen on cell surface) of CTCs, a monoclonal antibody specifically binding to the cell antigen, or the like have heretofore been disclosed as to the detection of CTCs. However, these CTC detection or measurement techniques have the restriction that they are applicable in the case where target cancer such as breast cancer, colorectal cancer, or prostate cancer has already been determined. Thus, a technique of effectively detecting or measuring cancer CTCs even for a wide range of cancers in the case where target cancer cells cannot be determined yet has not been found yet.

On the other hand, methods for detecting CTCs without employing a cell marker (specific antigen on cell surface) of CTCs, a monoclonal antibody specifically binding to the cell antigen, or the like are also disclosed as methods for detecting or measuring CTCs. For example, in Japanese unexamined Patent Application Publication No. 2011-163830, disclosed is a CTC detection or measurement method of thoroughly performing the processes of condensation of CTCs from blood, staining of CTCs, and washing in one device using a microfluid device capable of capturing CTCs contained in a blood sample by a size-selective microcavity array having fine through-holes for CTC capture in which the pore size, the number of holes, and geometry have been controlled, and rapidly counting CTCs under an automatic fluorescence microscope or the like. Also, in Japanese unexamined Patent Application Publication No. 2013-36818, disclosed is a method of contacting a body fluid containing tumor cells with a blood cell separation material consisting of a nonwoven fabric made of polyester fiber, polypropylene fiber, or the like having a density of 2.0 × 10⁴ to 1.9 × 10⁵ and a fiber diameter of 1 µm to 15 µm, to capture the tumor cells, leukocytes and platelets, and separating or recovering a fraction rich in the captured tumor cells in the body fluid employing a separating solution consisting of physiological saline, a buffer solution, dextran, etc.

Furthermore, in Japanese unexamined Patent Application Publication No. 2014-224800, disclosed is a method for separating CTCs, etc. by providing a body and a cover that define a void therebetween, the void forming a separation element that segregates an inlet region and an outlet region of the void, the separation element defining a channel together with a surface of the void and separating CTCs which are larger particles from blood cells which are smaller particles, by allowing the smaller particles to pass through the channel and inhibiting the larger particles from passing through the channel as to particles that flow in the channel. These methods for separating or detecting CTCs physically separate or detect CTCs present in a body fluid and do not employ a cell marker (specific antigen on cell surface) of CTCs, a monoclonal antibody specifically binding to the cell antigen, or the like. Therefore, these methods are applicable to the separation or detection of CTCs of cancer even if intended cancer cells cannot be determined yet for the cancer. Unfortunately, it is difficult to reliably separate or detect CTCs present at a level of a trace amount in a body fluid by the methods as described above.

The obtainment of cancer cells including CTCs is indispensable factor for cancer research, in particular, diagnosis or prognosis assessment based on gene analysis of cancer, the selection of effective cancer chemotherapy, etc. Also, the obtainment of cancer cells is a key technique for vaccine development in cancer immunotherapy, and further for order-made vaccine development in individualized medicine. Furthermore, for the adoptive cell transfer therapy of individual medicine, CTCs are also indispensable as a stimulatory factor in the induction of killer cells responsible for patient's own specific cytotoxic response to cancer, or as autologous cancer target cells upon measuring the cytotoxicity of the induced killer cells. Also, the obtainment of cancer stem cells whose presence has received attention as a factor of postoperative recurrence and metastasis in recent years provides revolutionary means to cancer stem cell research and the development of control techniques thereof, which are urgently necessary at present.

For the obtainment of cancer cells in medical practice, there have existed so far only methods having certain risks, such as biopsy, obtainment from operation materials, operation highly invasive to organisms and metastasis enhancement or dissemination associated therewith. Thus, the development of a method for separating/obtaining CTCs safely, conveniently, and in a short time from, for example, peripheral blood while avoiding these risks makes a very great contribution to basic research and clinical research on cancer. Thus, CTC separation/obtainment has important meaning.

Although 50 or more years have passed since cancer metastasis was found to occur typically in a hematogenous manner, not in a lymphogenous manner (Cancer Res. 11, 648-651, 1951; and CANCER JULY-AUGUST, Vol. 13: 674-676, 1960), a method for stably collecting cancer cells from peripheral blood has not been developed yet. As described above, methods of detecting or amplifying, with an antibody, a very trace amount of a cell fragment derived from a cancer cell present in peripheral blood, and studying the presence or absence of cancer have been known as methods for particular objects. However, these methods are means applicable only to cancer cells in which an objective substance to be bound by the antibody has already been determined. Thus, they are means inapplicable to cancer cells whose free substance is unknown. The same holds true for methods of amplifying a cancer cell-derived gene fragment by PCR and studying the presence or absence of cancer. Specifically, these methods assess the presence or absence of cancer cells by amplifying an already revealed gene segment. The weak point thereof is that the methods neither use an unknown gene nor can be applied to malignantly transformed cells. Meanwhile, for the obtainment of cancer cells, biopsy or a method invasive to organisms for collection from operation materials is possible as a method for overcoming this weak point. However, such obtainment of cancer cells is not ethically accepted because collection from a metastatic lesion might incur further dissemination of cancer cells, though biopsy or surgical collection from a primary lesion is feasible and acceptable. Moreover, it is impossible to deal with early-stage cancer whose cancer type has not been determined yet.

The detection or separation/obtainment of cancer cells by the detection or separation/obtainment of CTCs has very important meaning for the needs of medical practice for the detection or separation/obtainment of cancer cells as mentioned above. However, a method for reliably and stably detecting or separating/obtaining CTCs or CTSCs present in trace amounts in a biological circulating body fluid such as blood or lymph, even in the state where the cancer type of the tumor cells cannot be determined yet has not been developed yet under the present circumstances. Thus, it is a very important challenge to a basic or clinical approach of cancer to develop a method for safely, conveniently, reliably and stably detecting or separating/obtaining CTCs or CTSCs present in trace amounts from a biological circulating body fluid such as blood or lymph, for example, blood such as peripheral blood, even as to cancer cells whose cancer type has not been determined yet.

### Prior Art Documents

### Patent Documents

Patent document 1: Japanese unexamined Patent Application Publication No. 2007-178193
Patent document 2: Japanese unexamined Patent Application Publication No. 2011-163830
Patent document 3: Japanese unexamined Patent Application Publication No. 2012-103077
Patent document 4: Japanese unexamined Patent Application Publication No. 2013-36818
Patent document 5: Japanese unexamined Patent Application Publication No. 2014-39480
Patent document 6: Japanese unexamined Patent Application Publication No. 2014-105159
Patent document 7: Japanese unexamined Patent Application Publication No. 2014-112094
Patent document 8: Japanese unexamined Patent Application Publication No. 2014-224800
Patent document 9: Japanese unexamined Patent Application Publication (Translation of PCT Application) No. 2002-503814
Patent document 10: Japanese unexamined Patent Application Publication (Translation of PCT Application) No. 2009-537021
Patent document 11: WO99/41613
Patent document 12: WO2007/133465

### Non-patent Documents

Non-patent document 1: Estimated using the method by Wum LM et al., Estimating lifetime and age-conditional probabilities of developing cancer, Lifetime Data Anal., 1998, 4: 169-186
Non-patent document 2: Breast Cancer Res Treat 2010, 124: 403-412
Non-patent document 3: New Engl. J Med 2004, 351: 781-791
Non-patent document 4: Clin Cancer Res 2008, 14: 6302-6309
Non-patent document 5: J. Clin Oncol 2008, 26: 3213-3221
Non-patent document 6: Clin Cancer Res 2008, 14: 7004-7010
Non-patent document 7: Proc (Bayl Univ Med Cent) 2008, 21: 127-132
Non-patent document 8: J Translational-medicine 2012, 10: 138: 1-20
Non-patent document 9: Proc. Natl. Acad. Sci, USA, 95: 4589-4594, 1998
Non-patent document 10: Cancer Res. 11, 648-651, 1951
Non-patent document 11: CANCER JULY-AUGUST, Vol. 13: 674-676, 1960

### Summary of the Invention

### Object to be Solved by the Invention

An object of the present invention is to develop a method for detecting or separating/obtaining CTC and/or CTSC, which is capable of reliably and stably detecting or separating/obtaining CTC (circulating tumor cell) and CTSC (circulating tumor stem cell) present in trace amounts in a biological circulating body fluid such as blood or lymph, even in the state where the cancer type of the tumor cells cannot be determined yet and the state where the tumor cells are present in trace amounts in the biological circulating body fluid.

### Means to Solve the Object

To attain the object, the present inventor has conducted diligent studies on a method for detecting or separating/obtaining CTC and CTSC, which is capable of reliably and stably detecting or separating/obtaining CTC and CTSC present in trace amounts in a biological circulating body fluid such as blood or lymph, even in the state where the cancer type of the tumor cells cannot be determined yet and the state where the tumor cells are present in trace amounts in the biological circulating body fluid, and, during this course, completed the present invention by finding that in the method for detecting or separating/obtaining CTC and/or CTSC, CTC and/or CTSC sparsely present in a sample can be amplified and reliably and stably detected or separated/obtained by providing the detection or separation/obtainment step with a CTC and/or CTSC proliferation step employing a culture medium consisting of a serum-free culture medium for CTC and/or CTSC (circulating tumor cell and/or circulating tumor stem cell) proliferation.

Specifically, the present invention provides a method for detecting or separating/obtaining CTC and/or CTSC in a biological circulating body fluid employing a cell proliferation method, comprising the following treatment steps (1) to (4):
(1) a first step of pretreating a sample from the biological circulating body fluid to obtain a mononuclear cell phase;
(2) a second step of providing a well plate in which a culture medium consisting of a serum-free cell growth medium for CTC and/or CTSC has been injected and seeding thereto the mononuclear cell obtained in the first step, followed by incubation;
(3) a third step of removing the culture medium from a well of the plate obtained by the incubation in the second step; and
(4) a fourth step of detecting or separating/obtaining an adherent tumor cell attached to the well of the plate after the third step.

By the method for detecting or separating/obtaining CTC and/or CTSC employing a cell proliferation method according to the present invention, CTC and/or CTSC sparsely present in a sample such as a circulating body fluid can be amplified and reliably and stably detected or separated/obtained. The method for detecting or separating/obtaining CTC and/or CTSC according to the present invention can be performed with a biological circulating body fluid as a sample. Examples of the sample from the biological circulating body fluid can include a blood sample from peripheral blood as a sample that can be operated most easily and an effective sample.

In the method for detecting or separating/obtaining CTC and/or CTSC according to the present invention, the step of obtaining a mononuclear cell phase from the biological circulating body fluid, which is the first step involves pretreating a sample from the biological circulating body fluid, and examples thereof can include a treatment for the removal of a liquid component and a non-cellular component of blood cells or the like contained in the biological circulating body fluid.

In the method for detecting or separating/obtaining CTC and/or CTSC according to the present invention, the second step of seeding and incubating the mononuclear cells obtained in the first step consists of providing a well plate in which a culture medium consisting of a serum-free cell growth medium for CTC and/or CTSC has been injected, and seeding thereto the mononuclear cells obtained in the first step, followed by incubation. Examples of the culture medium consisting of a serum-free cell growth medium for CTC and/or CTSC can include an AIM-V-based culture medium which is a serum-free culture medium for cell proliferation. The employment of the culture medium enables CTC and/or CTSC to be proliferated in a short period in the incubation step in the method for detecting or separating/obtaining CTC and/or CTSC in a biological circulating body fluid employing a cell proliferation method, and enables CTC and/or CTSC present in trace amounts in the sample to be proliferated or amplified and reliably and stably detected or separated/obtained. An AIM-V medium, or a culture medium in which one or more components selected from autologous serum of a subject, healthy individual-derived AB serum and palmitic acid or a salt thereof have been added to the AIM-V culture medium can be employed as the an AIM-V-based culture medium. The conditions for the incubation in the second step can be appropriately set as compatible conditions as to a proliferation temperature and a proliferation period thereof. The incubation can be performed most suitably with 37°C and 3 to 7 days as standard conditions of a proliferation temperature and a proliferation period. Also, the conditions for the incubation in the second step can be conditions within an incubator adjusted to 5% CO₂.

Specifically, the culture medium consisting of a serum-free cell growth medium for CTC and/or CTSC, employed in the method for detecting or separating/obtaining CTC and/or CTSC according to the present invention can employ an AIM-V-based culture medium. The AIM-V-based culture medium can employ the AIM-V culture medium itself as a basic culture medium. By employing a culture medium in which one or more components selected from autologous serum of a subject, healthy individual-derived AB serum and palmitic acid or a salt thereof have been added to the AIM-V culture medium, an effect of inducing CTC can be enhanced, and an effect of more efficiently and stably detecting or separating CTC and/or CTSC can be obtained. Particularly, palmitic acid or a salt thereof can be mentioned as an additive component that enables CTC and/or CTSC to be safely and stably detected or separated.

In the method for detecting or separating/obtaining CTC and/or CTSC according to the present invention, the third step of removing the culture medium from a well of the plate obtained by the incubation in the second step consists of a treatment to remove the culture medium by appropriate means from cultures obtained by the incubation for a predetermined time in the well plate. In the method for detecting or separating/obtaining CTC and/or CTSC according to the present invention, the fourth step of detecting or separating/obtaining an adherent tumor cell attached to the well of the plate after the third step consists of directly detecting an adherent tumor cell attached to the well of the plate employing detection means such as microscopic examination, dye staining, or antigen-antibody staining, or separating an adherent tumor cell attached to the well of the plate and obtaining it as a cancer cell for various detections.

The background to the construction of the method for detecting or separating/obtaining CTC and/or CTSC according to the present invention will be described. For reliably and stably detecting or separating/obtaining CTC and/or CTSC present in trace amounts in a biological circulating body fluid, it is necessary to effectively proliferate and amplify, in a short period, CTC and/or CTSC present in trace amounts in a sample. Furthermore, a challenge for constructing the method for detecting or separating/obtaining CTC and/or CTSC employing a cell proliferation method is to search for a serum-free cell growth medium for CTC and/or CTSC that permits proliferation of CTC and CTSC. Accordingly, the present inventor conducted diligent studies to attain the object of the present invention and, during this course, compared an AIM-V culture medium (AIM-V) as a serum-free cell growth medium with reference to a RPMI-1640 culture medium (RPMI-1640), which is a standard culture medium for peripheral blood mononuclear cells, as to the separation and obtainment of CTC. Specifically, the present inventor added 5% fetal bovine serum (FBS) or autologous serum (AS)of a peripheral blood donor to each of these culture media for culture, and studied the presence or absence and an effect of CTC separation and obtainment among the culture media. As a result, it was demonstrated that: AIM-V is useful for the separation and obtainment of CTCs and/or CTSCs; and AS addition potentiates the effect thereof.

On the basis of the results using the culture media described above, various candidates, LPS, ConA, PHA, IL-1α, and IL-1β, including palmitic acid were further studied as novel CTC-separating/obtaining factors, and searched for the safest and most efficient factor, in order to avoid increase in burdens on patients by autologous serum collection and eliminate the influence of variation in serum components among the disease states of subjects. Although a CTC-inducing effect was also observed by LPS during the course of this study, LPS is a substance having very strong toxicity and was therefore judged as being inappropriate as a reagent for safety reasons in light of various purposes of research and clinical application technique development after CTC separation and excluded from subsequent experiments. As a result, it was demonstrated that for CTC obtainment, palmitic acid is very useful as a novel factor that can safely and stably disperse CTCs, even if autologous serum, which is an unstable factor in terms of its supply or properties (carrier of a virus that must not be allowed to infect others, etc.), is eliminated. In conclusion, the present inventor has demonstrated that an AIM-V culture medium, or a culture medium in which one or more components selected from autologous serum of a subject, healthy individual-derived AB serum and palmitic acid or a salt thereof have been added to the AIM-V culture medium, particularly, a culture medium in which palmitic acid or a salt thereof has been added, is useful for the stable isolation of CTCs, reaching the completion of the present invention.

Specifically, the present invention provides the following items:
[1] A method for detecting or separating/obtaining a circulating tumor cell and/or a circulating tumor stem cell in a biological circulating body fluid, comprising the following treatment steps (1) to (4):
   (1) a first step of pretreating a sample from the biological circulating body fluid to obtain a mononuclear cell phase;
   (2) a second step of providing a well plate in which a culture medium consisting of a serum-free cell growth medium for circulating tumor cell and/or circulating tumor stem cell has been injected, and seeding thereto the mononuclear cells obtained in the first step, followed by incubation;
   (3) a third step of removing the culture medium from a well of the plate obtained by the incubation in the second step; and
   (4) a fourth step of detecting or separating/obtaining an adherent tumor cell attached to the well of the plate after the third step.
[2] The method for detecting or separating/obtaining a circulating tumor cell and/or a circulating tumor stem cell in a biological circulating body fluid according to [1] above, wherein in the second step, the culture medium consisting of a serum-free cell growth medium for circulating tumor cell and/or circulating tumor stem cell, for seeding and incubating the mononuclear cells obtained in the first step is an AIM-V-based culture medium.
[3] The method for detecting or separating/obtaining a circulating tumor cell and/or a circulating tumor stem cell in a biological circulating body fluid according to [1] or [2] above, wherein in the second step, the AIM-V-based culture medium for seeding and incubating the mononuclear cells obtained in the first step is an AIM-V culture medium, or a culture medium in which one or more components selected from autologous serum of a subject, healthy individual-derived AB serum and palmitic acid or a salt thereof have been added to the AIM-V culture medium.
[4] The method for detecting or separating/obtaining a circulating tumor cell and/or a circulating tumor stem cell in a biological circulating body fluid according to any one of [1] to [3] above, wherein the sample from the biological circulating body fluid is a blood sample from peripheral blood.
[5] The method for detecting or separating/obtaining a circulating tumor cell and/or a circulating tumor stem cell in a biological circulating body fluid according to any one of [1] to [3] above, wherein the pretreatment of the sample from the biological circulating body fluid in the first step is removal of a liquid component and a non-cellular component contained in the biological circulating body fluid.
[6] The method for detecting or separating/obtaining a circulating tumor cell and/or a circulating tumor stem cell in a biological circulating body fluid according to any one of [1] to [5] above, wherein the incubation in the second step is performed with 37°C and 3 to 7 days as standard conditions of a proliferation temperature and a proliferation period.
[7] The method for detecting or separating/obtaining a circulating tumor cell and/or a circulating tumor stem cell in a biological circulating body fluid according to [6] above, wherein the incubation in the second step is performed in an incubator adjusted to 5% CO₂.

### Effect of the Invention

The present invention provides a method for detecting or separating/obtaining CTC, which is capable of reliably and stably detecting or separating/obtaining a circulating tumor cell and a circulating tumor stem cell present in trace amounts in a biological circulating body fluid such as blood or lymph, even in the state where the cancer type of the tumor cells cannot be determined yet and the state where the tumor cells are present in trace amounts in the biological circulating body fluid. The method for detecting or separating/obtaining CTC according to the present invention enables not only CTCs but CTSCs to be detected or separated and provides means useful for the basic elucidation of cancer or a clinical approach therefor.

Specifically, the efficient and stable isolation of CTCs enabled by the present invention offers the possibility of revolutionary headway in research on cancer. Cancer cells or moreover CTSCs have been unable to be efficiently and stably detected or separated or isolated so far from peripheral blood or the like. This has been a leading cause of a delay in CTC research. In light of this, the technique of the present invention has provided a foundation on which to drastically promote CTC research. The present invention enables basic or clinical research on CTCs to be pursued and can be expected to kick-start the development of a CTC control technique.

Provided that basic research using the present invention is pursued, it is possible to genetically study the cytological diverse aspects of CTCs at the level of a substance such as a gene as an entity. This opens a new vista of development of novel drugs related to proliferation control thereof and, immunologically, novel drugs such as cytocidal antibodies against CTCs. Also, this clinically permits comparison with stage classification of cancer patients, comparison with histological classification, and further, seeking of correlation with various obtained indexes. Furthermore, it can be expected that a solid scientific foundation is established even for world's first diagnosis, treatment, or prognosis evaluation or assessment, because relationship to the effect of treatment, etc. can also be clarified. Thus, the present invention makes a very great contribution to research and development such as basic elucidation of cancer or clinical application thereto.

### Brief Description of Drawings

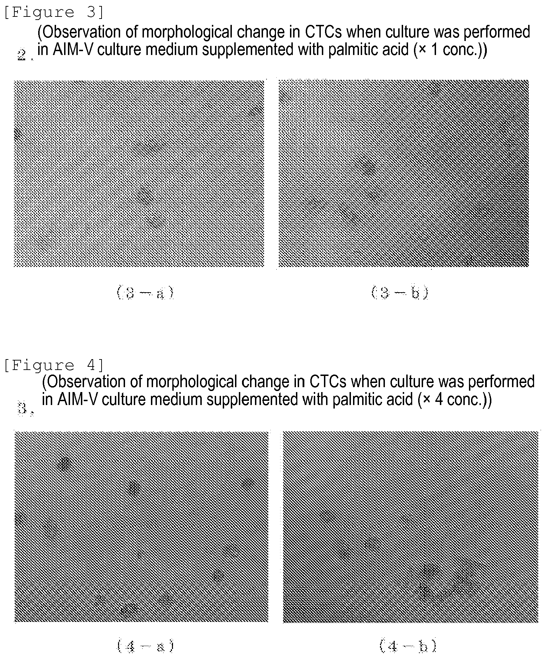

[Figure 1] Figure 1 is a diagram showing results (photograph) of microscopically observing one obtained in a CTC obtainment test using an AIM-V culture medium and a RPMI-1640 culture medium as culture media in a selection test of an efficient culture medium for cancer cell (CTC) obtainment. Figure (1-a) shows results of obtaining CTCs using the AIM-V culture medium, and Figure (1-b) shows results of obtaining CTCs in the case of using the RPMI-1640 culture medium.
[Figure 2] Figure 2 is a diagram showing a microscopic image of CTCs obtained by culture as to results of "observing morphological change in CTCs obtained by culture using an AIM-V culture medium alone" in a confirmation test of morphological change in cancer cells (CTCs) obtained in a culture medium for CTC proliferation in Examples of the present invention. In the figures, (2-a) and (2-b) show results of studying CTCs of different patients (microscopic photographs of cancer cells (CTCs) from different patients).

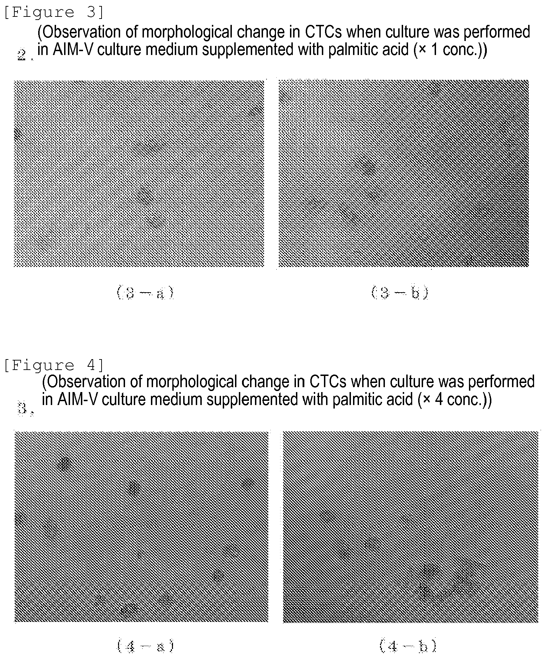

[Figure 3] Figure 3 is a diagram showing a microscopic image of CTCs obtained by culture as to results of "observing morphological change in CTCs obtained by culture using an AIM-V culture medium supplemented with palmitic acid (× 1 conc.)" in a confirmation test of morphological change in cancer cells (CTCs) obtained in a culture medium for CTC proliferation in Examples of the present invention. In the figures, (3-a) and (3-b) show results of studying CTCs of different patients (microscopic photographs of cancer cells (CTCs) from different patients).
[Figure 4] Figure 4 is a diagram showing a microscopic image of CTCs obtained by culture as to results of "observing morphological change in CTCs obtained by culture using an AIM-V culture medium supplemented with palmitic acid (× 4 conc.)" in a confirmation test of morphological change in cancer cells (CTCs) obtained in a culture medium for CTC proliferation in Examples of the present invention. In the figures, (4-a) and (4-b) show results of studying CTCs of different patients (microscopic photographs of cancer cells (CTCs) from different patients).

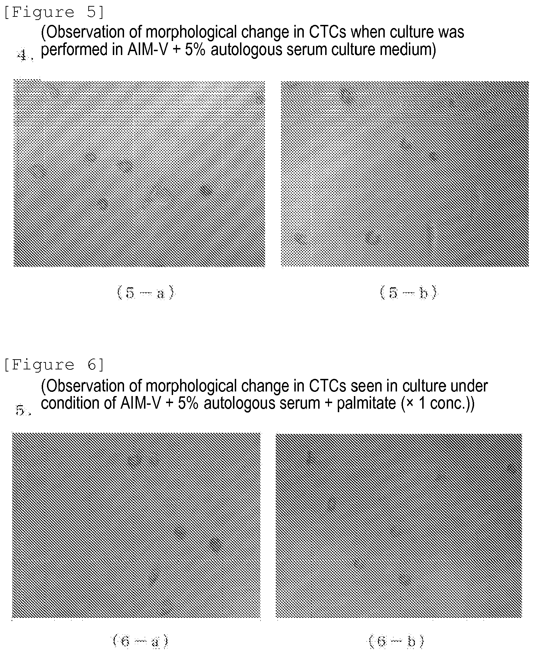

[Figure 5] Figure 5 is a diagram showing a microscopic image of CTCs obtained by culture as to results of "observing morphological change in CTCs obtained by culture using an AIM-V + 5% autologous serum culture medium" in a confirmation test of morphological change in cancer cells (CTCs) obtained in a culture medium for CTC proliferation in Examples of the present invention. In the figures, (5-a) and (5-b) show results of studying CTCs of different patients (microscopic photographs of cancer cells (CTCs) from different patients).
[Figure 6] Figure 6 is a diagram showing a microscopic image of CTCs obtained by culture as to results of "observing morphological change in CTCs obtained by culture using a culture medium under a condition of AIM-V + 5% autologous serum + palmitic acid (× 1 conc.)" in a confirmation test of morphological change in cancer cells (CTCs) obtained in a culture medium for CTC proliferation in Examples of the present invention. In the figures, (6-a) and (6-b) show results of studying CTCs of different patients (microscopic photographs of cancer cells (CTCs) from different patients).

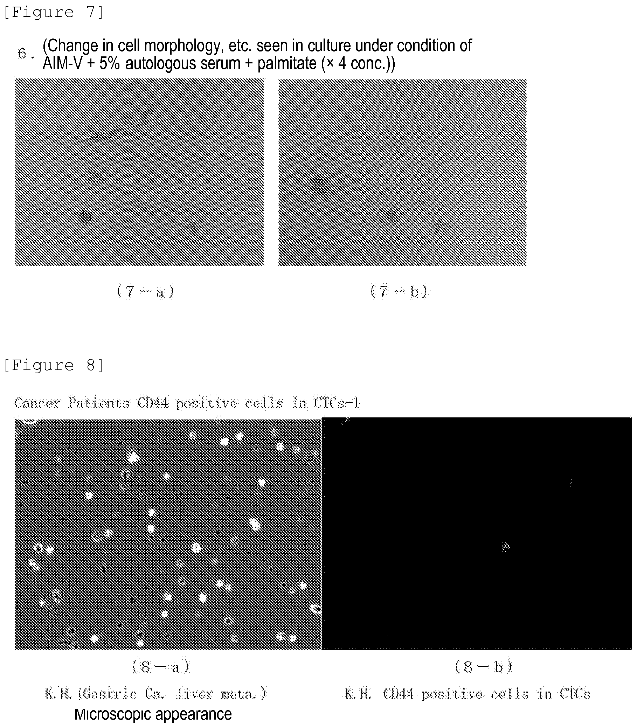

[Figure 7] Figure 7 is a diagram showing a microscopic image of CTCs obtained by culture as to results of "observing morphological change in CTCs obtained by culture using a culture medium under a condition of AIM-V + 5% autologous serum + palmitic acid (× 4 conc.)" in a confirmation test of morphological change in cancer cells (CTCs) obtained in a culture medium for CTC proliferation in Examples of the present invention. In the figures, (7-a) and (7-b) show results of studying CTCs of different patients (microscopic photographs of cancer cells (CTCs) from different patients).
[Figure 8] Figure 8 is a diagram showing a microscopic photograph (a) and a fluorescence microscopic photograph (b) of CTCs separated and obtained from patient K.H. (Gastric Ca.: gastric cancer, liver meta.: liver metastasis) in "confirmation and identification of CTCs using a membrane antigen CD44" in a confirmation and identification test using the cell membrane antigen of CTCs separated and obtained from the peripheral blood of the patient in Examples of the present invention.

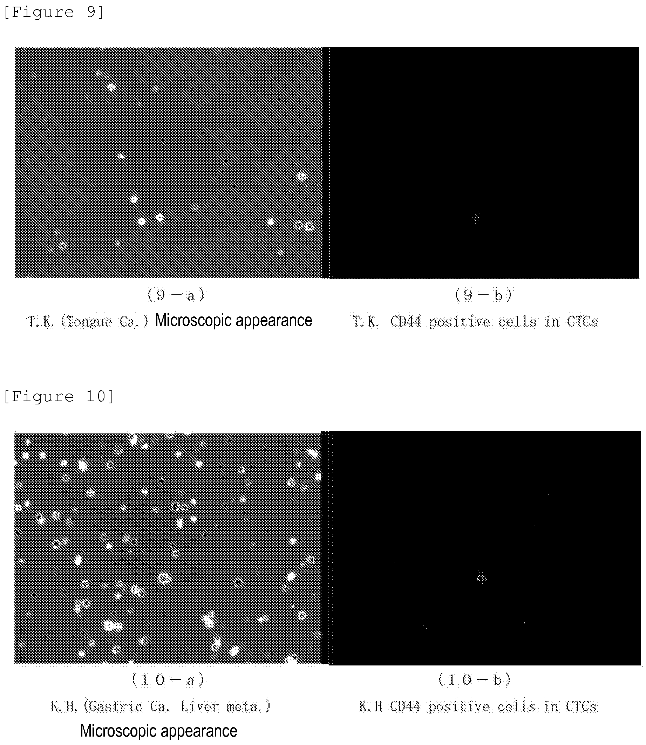

[Figure 9] Figure 9 is a diagram showing a microscopic photograph (a) and a fluorescence microscopic photograph (b) of CTCs separated and obtained from patient T.K. (Tongue Ca.: tongue cancer) in "confirmation and identification of CTCs using a membrane antigen CD44" in a confirmation and identification test using the cell membrane antigen of CTCs separated and obtained from the peripheral blood of the patient in Examples of the present invention.
[Figure 10] Figure 10 is a diagram showing a microscopic photograph (a) and a fluorescence microscopic photograph (b) of CTCs separated and obtained from patient K.H. (Gastric Ca.: gastric cancer, liver meta.: liver metastasis in "confirmation and identification of CTCs using a membrane antigen CD44" in a confirmation and identification test using the cell membrane antigen of CTCs separated and obtained from the peripheral blood of the patient in Examples of the present invention.

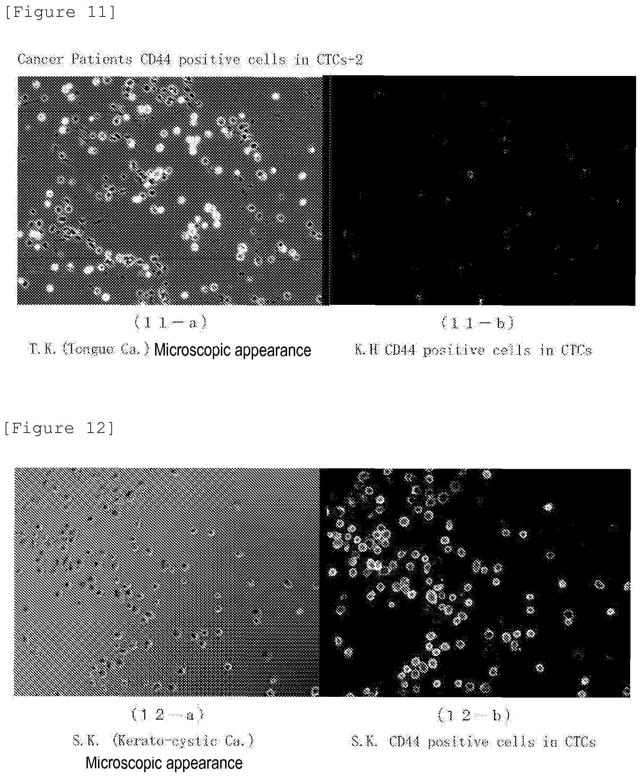

[Figure 11] Figure 11 is a diagram showing a microscopic photograph (a) and a fluorescence microscopic photograph (b) of CTCs separated and obtained from patient T.K. (Tongue Ca.: tongue cancer) in "confirmation and identification of CTCs using a membrane antigen CD44" in a confirmation and identification test using the cell membrane antigen of CTCs separated and obtained from the peripheral blood of the patient in Examples of the present invention.
[Figure 12] Figure 12 is a diagram showing a microscopic photograph (a) and a fluorescence microscopic photograph (b) of CTCs separated and obtained from patient S.K. (Kerato-cystic Ca.: keratocystic cancer) in "confirmation and identification of CTCs using a membrane antigen CD44" in a confirmation and identification test using the cell membrane antigen of CTCs separated and obtained from the peripheral blood of the patient in Examples of the present invention.

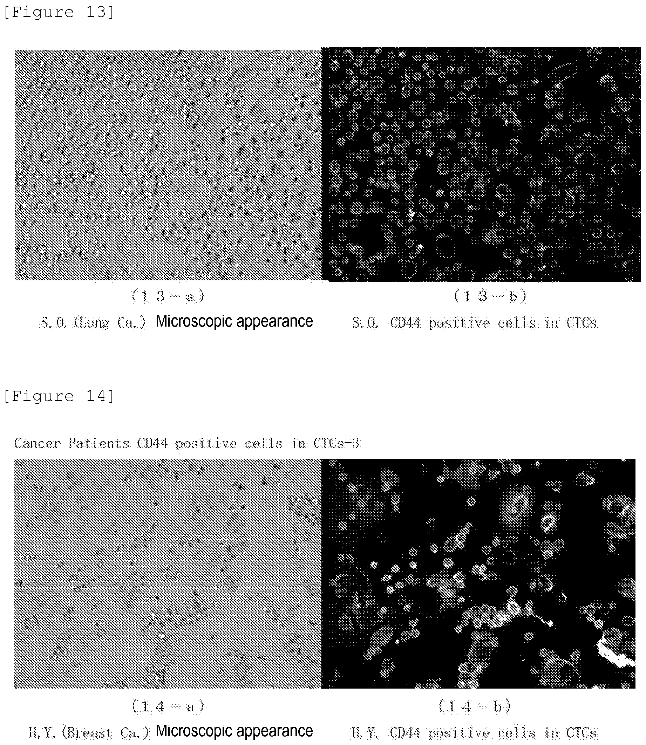

[Figure 13] Figure 13 is a diagram showing a microscopic photograph (a) and a fluorescence microscopic photograph (b) of CTCs separated and obtained from patient S.O. (Lung Ca.: lung cancer) in "confirmation and identification of CTCs using a membrane antigen CD44" in a confirmation and identification test using the cell membrane antigen of CTCs separated and obtained from the peripheral blood of the patient in Examples of the present invention.
[Figure 14] Figure 14 is a diagram showing a microscopic photograph (a) and a fluorescence microscopic photograph (b) of CTCs separated and obtained from patient H.Y. (Breast Ca.: breast cancer) in "confirmation and identification of CTCs using a membrane antigen CD44" in a confirmation and identification test using the cell membrane antigen of CTCs separated and obtained from the peripheral blood of the patient in Examples of the present invention.

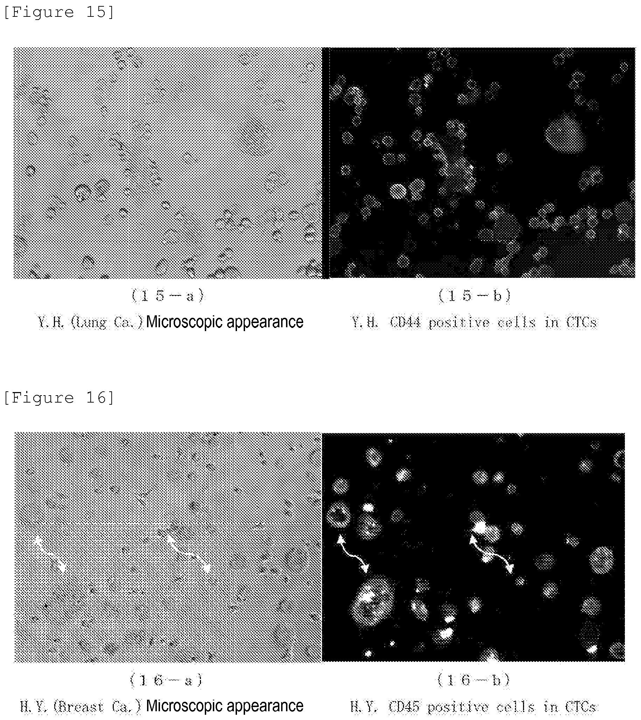

[Figure 15] Figure 15 is a diagram showing a microscopic photograph (a) and a fluorescence microscopic photograph (b) of CTCs separated and obtained from patient Y.H. (Lung Ca.: lung cancer) in "confirmation and identification of CTCs using a membrane antigen CD44" in a confirmation and identification test using the cell membrane antigen of CTCs separated and obtained from the peripheral blood of the patient in Examples of the present invention.
[Figure 16] Figure 16 is a diagram showing a microscopic photograph (a) and a fluorescence microscopic photograph (b) of CTCs separated and obtained from patient H.Y. (Breast Ca.: breast cancer) in "confirmation and identification of CTCs using a membrane antigen CD45" in a confirmation and identification test using the cell membrane antigen of CTCs separated and obtained from the peripheral blood of the patient in Examples of the present invention.

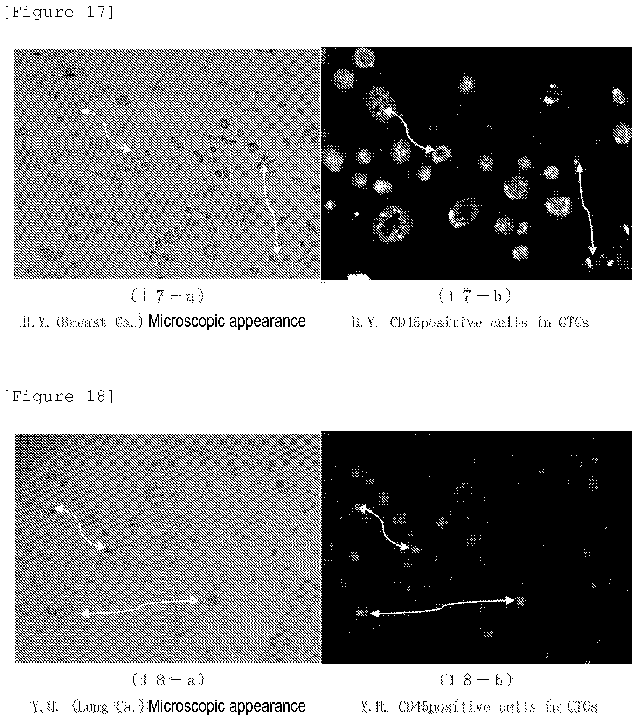

[Figure 17] Figure 17 is a diagram showing a microscopic photograph (a) and a fluorescence microscopic photograph (b) of CTCs separated and obtained from patient H.Y. (Breast Ca.: breast cancer) in "confirmation and identification of CTCs using a membrane antigen CD45" in a confirmation and identification test using the cell membrane antigen of CTCs separated and obtained from the peripheral blood of the patient in Examples of the present invention.
[Figure 18] Figure 18 is a diagram showing a microscopic photograph (a) and a fluorescence microscopic photograph (b) of CTCs separated and obtained from patient Y.H. (Lung Ca.: lung cancer) in "confirmation and identification of CTCs using a membrane antigen CD45" in a confirmation and identification test using the cell membrane antigen of CTCs separated and obtained from the peripheral blood of the patient in Examples of the present invention.

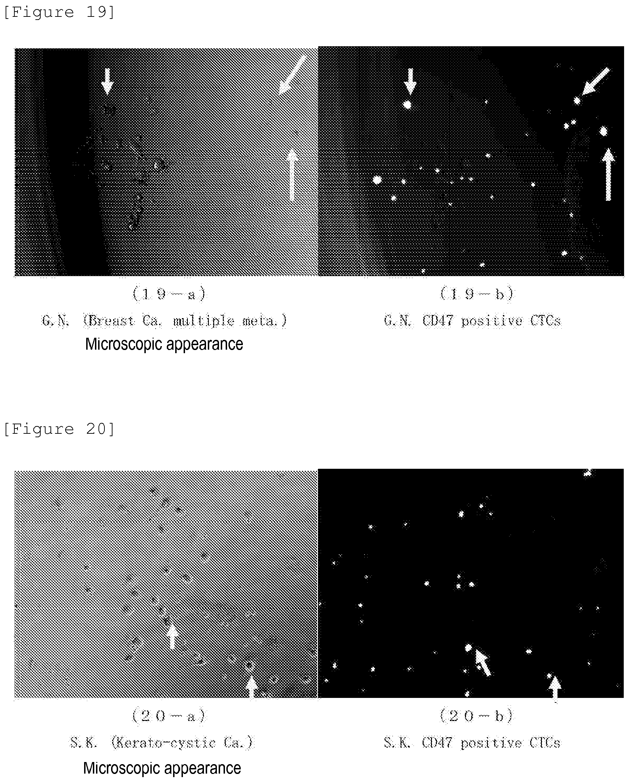

[Figure 19] Figure 19 is a diagram showing a microscopic photograph (a) and a fluorescence microscopic photograph (b) of CTCs separated and obtained from patient G.N. (Breast Ca.: breast cancer, multiple meta.: multiple metastasis) in "confirmation and identification of CTCs using a membrane antigen CD47" in a confirmation and identification test using the cell membrane antigen of CTCs separated and obtained from the peripheral blood of the patient in Examples of the present invention.
[Figure 20] Figure 20 is a diagram showing a microscopic photograph (a) and a fluorescence microscopic photograph (b) of CTCs separated and obtained from patient S.K. (Kerato-cystic Ca.: keratocystic cancer) in "confirmation and identification of CTCs using a membrane antigen CD47" in a confirmation and identification test using the cell membrane antigen of CTCs separated and obtained from the peripheral blood of the patient in Examples of the present invention.

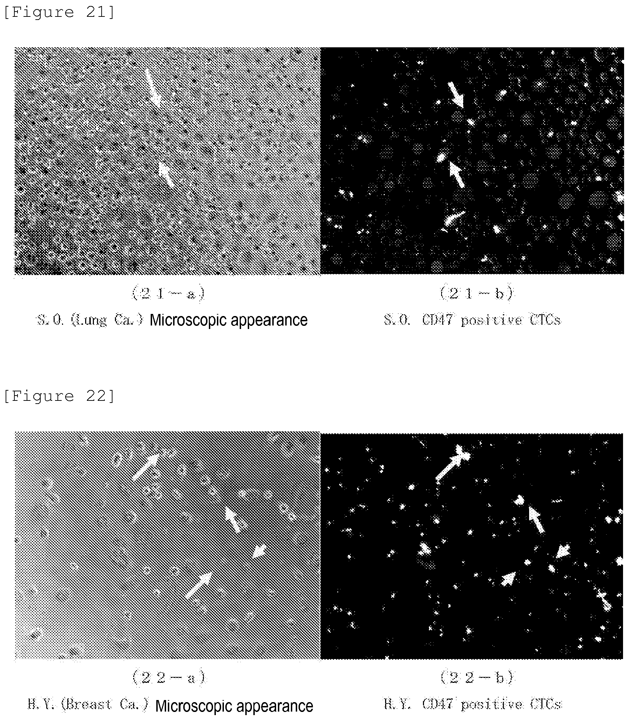

[Figure 21] Figure 21 is a diagram showing a microscopic photograph (a) and a fluorescence microscopic photograph (b) of CTCs separated and obtained from patient S.O. (Lung Ca.: lung cancer) in "confirmation and identification of CTCs using a membrane antigen CD47" in a confirmation and identification test using the cell membrane antigen of CTCs separated and obtained from the peripheral blood of the patient in Examples of the present invention.
[Figure 22] Figure 22 is a diagram showing a microscopic photograph (a) and a fluorescence microscopic photograph (b) of CTCs separated and obtained from patient H.Y. (Breast Ca.: breast cancer) in "confirmation and identification of CTCs using a membrane antigen CD47" in a confirmation and identification test using the cell membrane antigen of CTCs separated and obtained from the peripheral blood of the patient in Examples of the present invention.

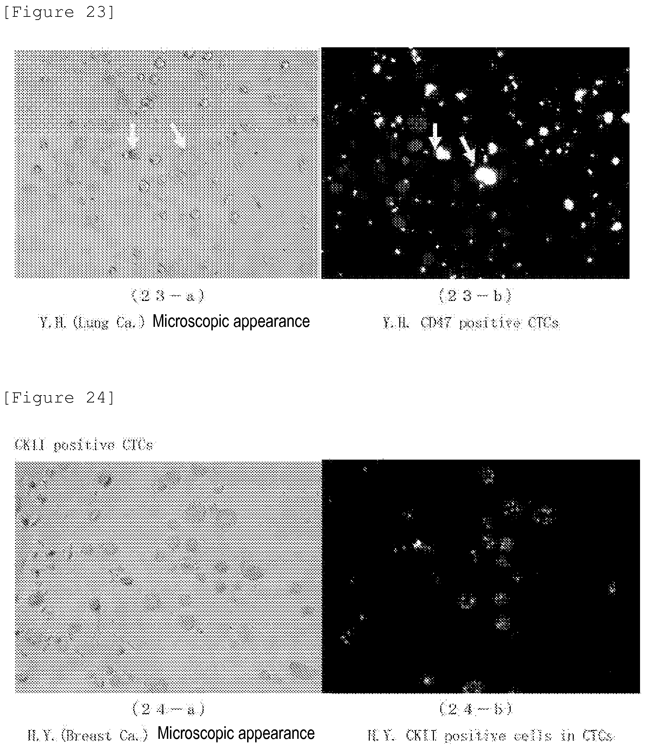

[Figure 23] Figure 23 is a diagram showing a microscopic photograph (a) and a fluorescence microscopic photograph (b) of CTCs separated and obtained from patient Y.H. (Lung Ca.: lung cancer) in "confirmation and identification of CTCs using a membrane antigen CD47" in a confirmation and identification test using the cell membrane antigen of CTCs separated and obtained from the peripheral blood of the patient in Examples of the present invention.
[Figure 24] Figure 24 is a diagram showing a microscopic photograph (a) and a fluorescence microscopic photograph (b) of CTCs separated and obtained from patient H.Y. (Breast Ca.: breast cancer) in "confirmation and identification of CTCs using a membrane antigen CKII" in a confirmation and identification test using the cell membrane antigen of CTCs separated and obtained from the peripheral blood of the patient in Examples of the present invention.

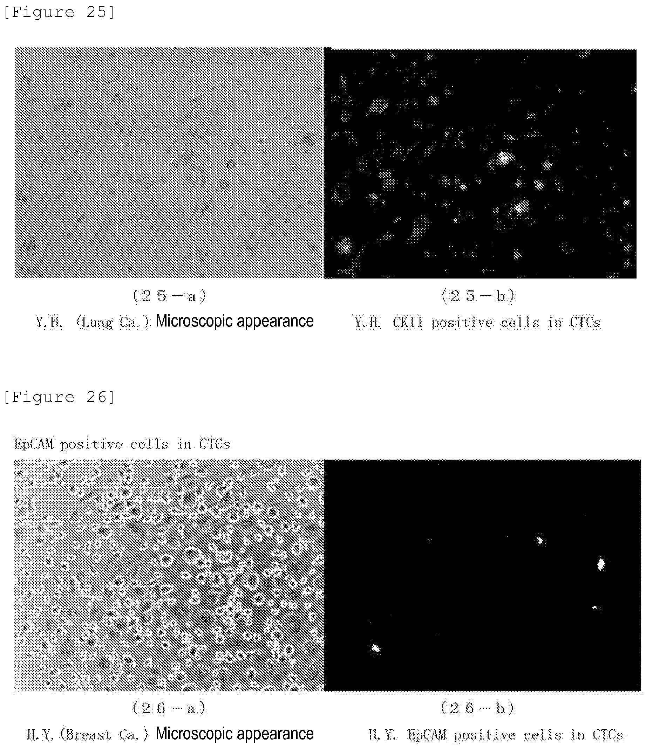

[Figure 25] Figure 25 is a diagram showing a microscopic photograph (a) and a fluorescence microscopic photograph (b) of CTCs separated and obtained from patient Y.H. (Lung Ca.: lung cancer) in "confirmation and identification of CTCs using a membrane antigen CKII" in a confirmation and identification test using the cell membrane antigen of CTCs separated and obtained from the peripheral blood of the patient in Examples of the present invention.
[Figure 26] Figure 26 is a diagram showing a microscopic photograph (a) and a fluorescence microscopic photograph (b) of CTCs separated and obtained from patient H.Y. (Breast Ca.: breast cancer) in "confirmation and identification of CTCs using a membrane antigen EpCAM" in a confirmation and identification test using the cell membrane antigen of CTCs separated and obtained from the peripheral blood of the patient in Examples of the present invention.

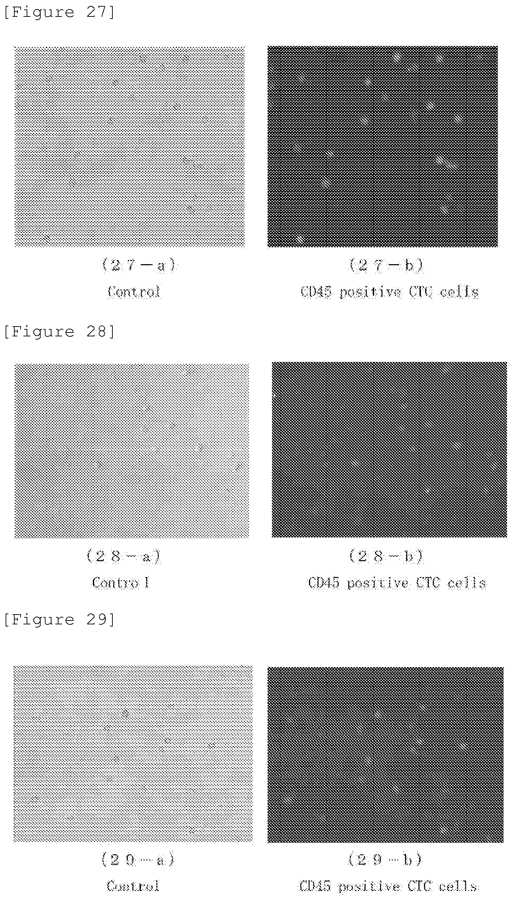

[Figure 27] Figure 27 is a diagram showing a microscopic photographic image (non-fluorescent emission image) of cells remaining in a CTC transplantation group and a fluorescent antibody microscope photograph (fluorescent emission image) thereof using a membrane antigen CD45, in a "confirmation test of the tumorigenicity or long-term survival of CTCs in nude mice" in Examples of the present invention. In the figures, (27-a) and (27-b) show the microscopic photograph (Control) and the fluorescent antibody microscope photograph (fluorescent emission image), respectively, of the cells remaining in the CTC transplantation group in the case where CTCs (HY-1) separated from a sample from patient H.Y. were transplanted in "Nude 1-1".
[Figure 28] Figure 28 is a diagram showing a microscopic photographic image (non-fluorescent emission image) of cells remaining in a CTC transplantation group and a fluorescent antibody microscope photograph (fluorescent emission image) thereof using a membrane antigen CD45, in a "confirmation test of the tumorigenicity or long-term survival of CTCs in nude mice" in Examples of the present invention. In the figures, (28-a) and (28-b) show the microscopic photograph (Control) and the fluorescent antibody microscope photograph (fluorescent emission image), respectively, of the cells remaining in the CTC transplantation group in the case where CTCs (KH-1) separated from a sample from patient K.H. were transplanted in "Nude 1-2".
[Figure 29] Figure 29 is a diagram showing a microscopic photographic image (non-fluorescent emission image) of cells remaining in a CTC transplantation group and a fluorescent antibody microscope photograph (fluorescent emission image) thereof using a membrane antigen CD45, in a "confirmation test of the tumorigenicity or long-term survival of CTCs in nude mice" in Examples of the present invention. In the figures, (29-a) and (29-b) show the microscopic photograph (Control) and the fluorescent antibody microscope photograph (fluorescent emission image), respectively, of the cells remaining in the CTC transplantation group in the case where CTCs (HY-1) separated from a sample derived from patient H.Y. were transplanted in "Nude 2-1".

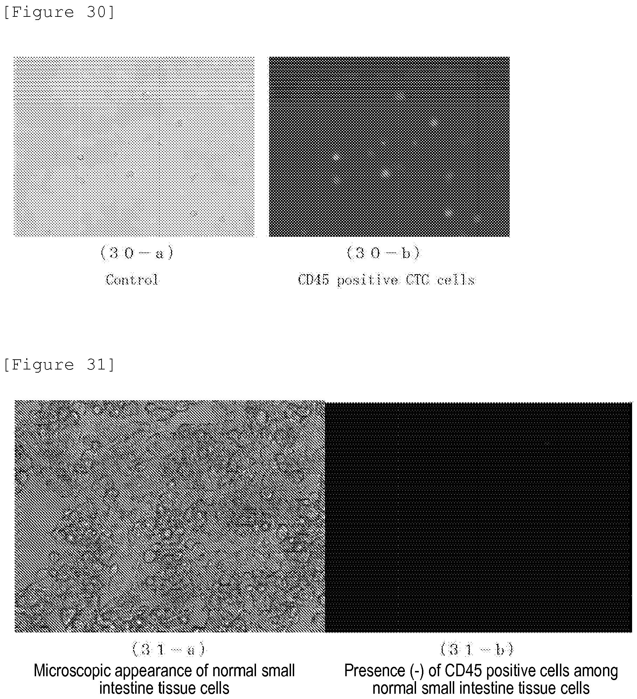

[Figure 30] Figure 30 is a diagram showing a microscopic photographic image (non-fluorescent emission image) of cells remaining in a CTC transplantation group and a fluorescent antibody microscope photograph (fluorescent emission image) thereof using a membrane antigen CD45, in a "confirmation test of the tumorigenicity or long-term survival of CTCs in nude mice" in Examples of the present invention. In the figures, (30-a) and (30-b) show the microscopic photograph (Control) and the fluorescent antibody microscope photograph (fluorescent emission image), respectively, of the cells remaining in the CTC transplantation group in the case where CTCs (KH-1) separated from a sample from patient K.H. were transplanted in "Nude 2-2".
[Figure 31] Figure 31 is a diagram showing a microscopic photographic image (a) and a fluorescent antibody microscope photograph (b) using a membrane antigen CD45 as to "normal small intestine tissue cells" of CTC-untransplanted control group nude mice in a "confirmation test of the tumorigenicity or long-term survival of CTCs in nude mice" in Examples of the present invention.

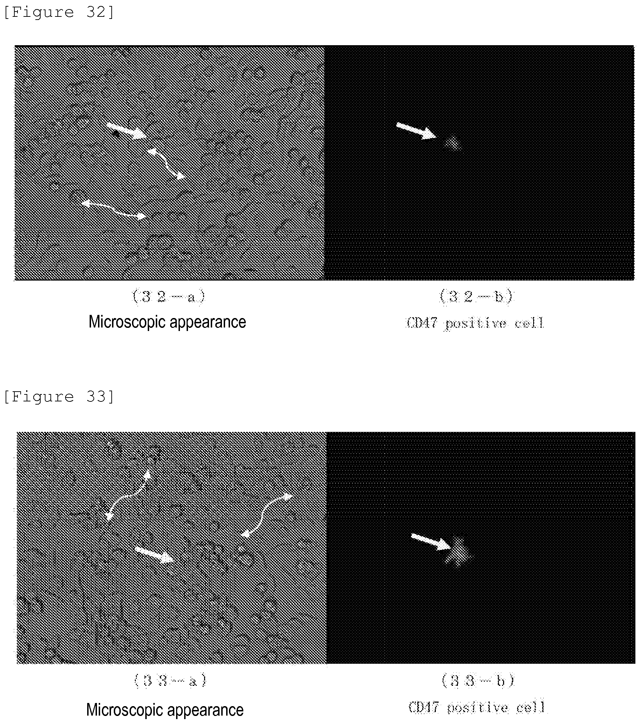

[Figure 32] Figure 32 is a photograph displaying, by a microscopic image of non-fluorochrome staining and a CD47 positive cell image by a fluorochrome staining method, results of proliferating and obtaining CTC cells using an established cancer cell line (UTC-8) as a sample, and then detecting CD47 positive cells using a cell membrane antigen CD47, in a detection test (I) of the CD47 positive cells (CTSCs) using the established cancer cell line (UTC-8). In Figure 32, (32-a) shows the microscopic image of non-fluorochrome staining, and (32-b) shows the CD47 positive cell image by the fluorochrome staining method.
[Figure 33] Figure 33 is a photograph displaying, by a microscopic image of non-fluorochrome staining and a CD47 positive cell image by a fluorochrome staining method, results of proliferating and obtaining CTC cells using an established cancer cell line (UTC-8) as a sample, and then detecting CD47 positive cells using a cell membrane antigen CD47 , in a detection test (II) of the CD47 positive cells (CTSCs) using the established cancer cell line (UTC-8). In Figure 33, (33-a) shows the microscopic image of non-fluorochrome staining, and (33-b) shows the CD47 positive cell image by the fluorochrome staining method.

### Mode of Carrying Out the Invention

The present invention provides a method for detecting or separating/obtaining CTC and/or CTSC in a biological circulating body fluid employing a proliferation method, comprising the following treatment steps (1) to (4):
(1) a first step of pretreating a sample from the biological circulating body fluid to obtain a mononuclear cell phase;
(2) a second step of providing a well plate in which a culture medium consisting of a serum-free cell growth medium for CTC and/or CTSC has been injected, and seeding thereto the mononuclear cells obtained in the first step, followed by incubation;
(3) a third step of removing the culture medium from a well of the plate obtained by the incubation in the second step; and
(4) a fourth step of detecting or separating/obtaining an adherent tumor cell attached to the well of the plate after the third step.

The method for detecting or separating/obtaining CTC and/or CTSC according to the present invention can be performed with a biological circulating body fluid as a sample. Examples of the sample from the biological circulating body fluid can include a blood sample from peripheral blood as a sample that can be operated most easily and an effective sample. As for the collection of the sample from the biological circulating body fluid for detecting or separating/obtaining CTC and/or CTSC, an organ isolated from the circulation of blood and a body fluid in an organism cannot exist in one individual. It is possible to isolate CTC and/or CTSC from every organ even if the presence of cancer therein cannot be macroscopically predicted. For example, human-derived CTCs have been successfully separated by subcutaneously or intracutaneously transplanting human-derived CTCs to the backs of nude mice, excising the spleen 3 months later, and carrying out separation operation from peripheral blood.

In the method for detecting or separating/obtaining CTC and/or CTSC in a biological circulating body fluid according to the present invention, the first step consists of the step of pretreating a sample from the biological circulating body fluid to obtain a mononuclear cell phase. The step involves pretreating a sample from the biological circulating body fluid, and examples thereof can include a treatment for the removal of a liquid component and a non-cellular component of blood cells or the like contained in the biological circulating body fluid. The treatment for the removal of a liquid component and a non-cellular component of blood cells or the like contained in the biological circulating body fluid is not particularly limited, and a publicly known method can be employed. Examples thereof for a blood sample include a method of separating and removing erythrocytes and leukocytes in blood by centrifugation (centrifugation method), a method of separating and removing erythrocytes and leukocytes in blood by utilizing cell density (density gradient centrifugation method), and a method of separating blood cells employing difference in cell size (separation method employing a filter). Particularly preferred examples of the method can include a density gradient centrifugation method. In this case, specifically, treatment conditions of a Ficoll-Isopaque density gradient centrifugation method can be employed.

In the method for detecting or separating/obtaining CTC and/or CTSC in a biological circulating body fluid according to the present invention, an AIM-V-based culture medium can be employed as the culture medium consisting of a serum-free cell growth medium for CTC and/or CTSC, employed in the incubation in the second step. The AIM-V culture medium is one developed as a culture medium for the proliferation of T cells and the like. The culture medium itself can be procured from commercially available products. The second step in the method for detecting or separating/obtaining CTC and/or CTSC in a biological circulating body fluid according to the present invention will be described. The second step consists of the step of providing a well plate in which an AIM-V-based culture medium which is a serum-free cell growth medium for cell proliferation has been injected, and seeding thereto the mononuclear cells obtained in the first step, followed by incubation. An AIM-V culture medium, or a culture medium in which one or more components selected from autologous serum of a subject, healthy individual-derived AB serum and palmitic acid or a salt thereof have been added to the AIM-V culture medium can be employed as the AIM-V-based culture medium. The conditions for the incubation in the second step can be appropriately set according to a situation. Preferably, the incubation can be performed under an intra-incubator condition of 5% CO₂ and under conditions of 37°C and 3 to 7 days. Particularly preferably, the incubation can be performed under an intra-incubator condition and culture temperature and culture period conditions of 5% CO₂, 37°C, and 7 days.

In the method for detecting or separating/obtaining CTC and/or CTSC in a biological circulating body fluid according to the present invention, the third step consists of the step of removing the culture medium from a well of the plate obtained by the incubation in the second step. The removal of the culture medium from the well of the plate can be performed by appropriate means.

In the method for detecting or separating/obtaining CTC and/or CTSC in a biological circulating body fluid according to the present invention, the fourth step consists of the step of detecting or separating/obtaining an adherent tumor cell attached to the well of the plate after the third step. In the fourth step, the adherent tumor cell attached to the well of the plate can be subjected directly to detection means employing detection means such as microscopic examination, dye staining, or antigen or antibody staining. Also, the adherent tumor cell attached to the well of the plate can be separated and obtained as a cancer cell for various detections.

In the method for detecting or separating/obtaining CTC and/or CTSC in a biological circulating body fluid according to the present invention, the cancer cells thus separated/obtained can be provided as cancer cell samples for research for cancer elucidation, clinical application, etc., such as cancer diagnosis, treatment, or prognosis evaluation or assessment.

The cells separated and obtained according to the present invention can be proved cancer cells by selecting a plurality of appropriate ones from criteria given below and confirming them. For example, a decision can be made by selecting (1) to (5), (7), and (9).

### (Confirmation criteria)

(1) No contact inhibition phenomenon is seen.
(2) The ability to form a colony within soft agar is exhibited (this method is a method that is applied to floating cells).
(3) Abnormal chromosomal morphology and capacity compared with the volume of the cells are seen under a microscope.
(4) The ability to divide beyond the Hayflick limit of approximately 50 times, which is reportedly the limit of the number of divisions under passage culture for human normal cells, is exhibited, i.e., the ability to proliferate permanently or immortalization is exhibited by passage culture for approximately half a year or longer, if cells divide two to three times a week.

### (Genetically or serologically)

(5) Antigens such as CD44, CD45, CD47, CKII, and EpCAM already revealed as cell surface markers of cancer stem cells (CTSCs) are possessed alone or in combination.
(6) The culture medium of the cells exhibits positive response to at least one or more of 24 types of markers generally reported as tumor markers, such as SCC for head and neck or esophageal squamous cell cancer, CA125 for lung cancer, CA15-3 for breast cancer, AFP and CEA for lung cancer, CEA and CA19-9 for pancreatic cancer, and CA19-9 for colorectal cancer, as compared with a control group.
(7) When total RNA is purified from the cells and subjected to gene analysis, the expression of cancer-related genes and cancer stem cell genes is significantly observed.

### (To observe tumorigenicity of obtained cells)

(8) When DNA is purified from the separated and obtained cancer cells and transferred to NIH3T3 cells, the contact inhibition phenomenon of the NIH3T3 cells disappears and the morphological expression of cancer cell traits is observed.
(9) When the cells are transplanted to immunodeficient nude mice or skid mice, the transplanted cells exhibit tumorigenicity. In addition, when proliferated tissues are recultured, the tissue cells proliferate.

As described above, for example, CTCs efficiently separated or detected by the method of the present invention from peripheral blood which can be operated most easily as a representative of a human biological tissue can be employed with the separation or detection results as an index for the comparative evaluation of an effect between before and after the practice of cancer therapy, and can contribute to the evaluation of prognosis. Also, the method of the present invention has enabled CTCs to be separated/obtained with high efficiency and can thereby promote the development of novel treatment techniques, etc., such as the development of drugs or the like effective for the cancer. Specifically, the present invention has enabled CTCs to be separated/obtained with high efficiency even for any cancer or unspecified cancer and thereby not only permits early detection of cancer or analysis or evaluation of prognosis, but can establish an index for evaluation before and after treatment on the basis of trends in the number or morphology of stably obtained CTCs to promote the development of drugs controlling CTCs or the development of treatment methods. Furthermore, the CTCs can be utilized as a material for establishing a stable foundation in basic or clinical CTC research.

Hereinafter, the present invention will be described further specifically with reference to Examples. However, the present invention is not limited by these Examples.

### Example 1

### [Experimental method]:

Examples of the present invention followed experimental methods given below. Specifically, all experimental samples (peripheral blood from various cancer patients who donated blood) were aseptically treated by procedures given below. In the case where infections by various viruses such as HBV and HCV were found in advance according to information from each cooperating facility, the treatment thereof was tested aside from a non-infected group and proper medical waste treatment was carried out by medical waste treatment professionals.

### (Collection of sample: blood collection)

1. A disposable 30 ml syringe for blood collection containing an appropriate amount of an anticoagulant heparin sodium (0.05 ml) was provided, and a disposable 30 ml blood collection syringe for serum procurement containing no heparin sodium was also provided. These syringes were respectively connected to insertion ports of L-type 180° three-way stopcock, while an appropriate 18-, 21- or 24-gauge butterfly needle was connected to the vascular vessel side to prepare for blood collection. 30 ml of blood per each sample was collected, and the following separation operation was carried out.

### (Purification of sample: blood cell separation)

2. For serum collection, the blood collection syringe barrel with a needle put thereon was placed at 37°C for 1 hour in a 5% CO₂ incubator, followed by centrifugation at 3,000 rpm (800 G) at 4°C for 30 minutes. The supernatant was collected as autologous serum (AS) and refrigerated at 4°C.
3. Meanwhile, the heparin blood collection tube intended to separate peripheral blood mononuclear cells (PBMCs) was thoroughly mixed by well rolling the blood collection tube after blood collection. Then, the same amount of (-)PBS was added therein and mixed to reduce blood viscosity. Then, a 5 ml aliquot thereof was slowly poured to the upper surface of F/I in each 15 ml tube provided in advance to which 5 ml of an agent for Ficoll-Isopaque (F/I) density gradient centrifugation was injected, so as not to be mixed into F/I. The tube was left standing. They were centrifuged at 3,000 rpm (1710 G) at 4°C for 30 minutes to obtain a mononuclear cell layer at the boundary between F/I and plasma. PBMCs were collected by aspiration therefrom, and (-)PBS was added thereto, followed by centrifugation at 1200 rpm (270 G) at 4°C for 15 minutes. This was repeated twice. After removal of the supernatant, a culture medium (RPMI-1640 or AIM-V) suitable for scheduled experimental conditions was added thereto, and the mixture was used in the following experiments.

### (Incubation in culture medium)

4. The obtained PBMCs were adjusted to 1 × 10⁴ cells/100 µl. Under an experimental condition of 3 wells per group on the basis of experimental design, the PBMCs were injected at 1×10⁴/100 µl (/well/96 well plate or 1 x 10⁴/25 µl/well/384 well plate) to each well of a 96-well plate (manufactured by BD Falcon/Becton, Dickinson and Company, 96 Well, Clear, Tissue Culture Treated Plate, Flat Bottom) or a 384-well plate (manufactured by BD Falcon/Becton, Dickinson and Company, 384 Well, Clear, Tissue Culture Treated Plate, Flat Bottom) and finished seeding of the cells. In the case of the culture medium alone, 100 µl of the culture medium was further injected to the well. As for a 5% AS group or groups using various additive drugs (LPS, IL-1α, IL-1β, and palmitic acid), 100 µl of each solution was provided and injected such that the allocation of concentrations was performed according to design. The total amount of the culture medium was set to 200 µl/well, and static culture was performed at 37°C for a predetermined time in an incubator under a 5% CO₂ condition to start an experiment.

### (Removal of culture medium: detection of adherent cells)

5. One week after the start of culture, the culture medium was removed by aspiration from each well, to which 100 µl of (-)PBS was then injected, and discarded after the inside of the well was well washed. This operation was repeated twice. Then, 100 µl of (-)PBS was injected again, and the adherent cells in each well were fractionated into normal PBMCs from their morphological features (size, shape, topology of intracellular granules, etc.) under a microscope, followed by calculation.

After the calculation of cell number of the 3 wells per group, an average value and standard deviation (Average ± SE) thereof was calculated. [These experiments were carried out plural times for the same patient, etc. over approximately 2 years. In spite of change in disease stage (stages I to IV) or disease state (trend of worsening or amelioration), the data was confirmed to always have reproducibility as response to a drug and exhibit an identical trend, as a whole as long as cancer was present, though decrease in obtained CTC count was seen. Thus, the data was adopted as formal data (see various data). It was also revealed that the detection of adherent cancer cells by this treatment resulted in one or less CTC in patients at a cancer stage regarded as being cured, as a matter of course. Furthermore, the adherent cells were not detected in individuals who seemed to be healthy.]

### (Detection of CTSCs by fluorescent staining)

6. The adherent cells obtained by the pretreatment were bound to specific antibodies against reported CTC (CTSC) surface antigens including CD44, CD45, CD47, CKII, EpCAM, then stained with a FITC- labeled secondary antibody, and observed under a fluorescence microscope. As a result, it was demonstrated that the adherent cells obtained by the treatment were CTCs (CTSCs) (see staining images).

### (Study on inducing effect of drug used)

7. Various culture conditions were set, and the CTC (CTSC)-inducing effects of the drugs used were studied. Each drug concentration, etc. used are described in tables about the results.

### (1) "Study on culture medium and additive drug - I"

As for a culture medium for the separation/obtainment of CTCs, an AIM-V culture medium (hereinafter, referred to as AIM-V) as a serum-free culture medium was compared with reference to a RPMI-1640 culture medium (hereinafter, referred to as RPMI-1640), which is a standard culture medium for peripheral blood mononuclear cells. Specifically, 5% fetal bovine serum (FBS), autologous serum (AS)of a peripheral blood donor, or AB-type serum was added to each of these culture media for culture, and the presence or absence and an effect of CTC separation and obtainment among the culture media were studied. As a result, it was demonstrated that: AIM-V is useful for the isolation of CTCs; AS addition potentiates the effect thereof; the effect of AB-type serum was slightly weaker than that of the AS serum; etc.

### (2) "Study on culture medium and additive drug - II"

On the basis of the results about the culture media described above, various candidates of additive components (LPS, ConA, PHA, IL-1α, and IL-1β) including palmitic acid were further studied as novel CTC-separating/obtaining factors, and search for the safest and most efficient factor was carried out, in order to avoid increase in burdens on patients by autologous serum collection, eliminate the influence of variation in serum components among the disease states of subjects, and remove essential instability possessed by AB-type serum such as uncertainty about whether or not to be truly cancer-bearing patient serum. Although a CTC-inducing effect was also slightly observed in LPS during the course of this study, LPS is a substance having very strong toxicity and was therefore judged as being inappropriate as a reagent for safety reasons in light of various purposes of research and clinical application technique development after CTC separation and excluded from subsequent experiments. As a result, it was confirmed that an AIM-V culture medium and the addition of palmitic acid produce the best consequence for the stable isolation of CTCs.

### (Sample to be tested)

8. In the experiments of the present invention, samples from terminal cancer patients with tongue cancer, malignant mandibular tumor, malignant lymphoma, breast cancer, lung cancer, gastric cancer, prostate cancer, uterine sarcoma, or the like, provided by hospitals were used as samples to be tested.

### Example 2

### [Selection test (I) of efficient culture medium for cancer cell (CTC) obtainment]

### <Culture medium to be tested>

The following culture media were provided and used as culture media to be tested.
(1) RPMI-1640 + 5% FBS
(2) RPMI-1640 + 5% Auto Serum (autologous serum)
(3) AIM-V (serum-free culture medium alone)
(4) AIM-V + 5% FBS
(5) AIM-V + 5% Auto Serum (autologous serum)

### <Experimental method>

Each culture medium was tested for cancer cell (CTC) yield efficiency (cancer cell detection accuracy) according to the experimental method described in Example 1 using the culture media described above.

### (Test 1: Study on CTCs-inducing ability using RPMI-1640)

RPMI-1640 was used as a culture medium, and this was used as a common culture medium. The culture medium was divided into a fetal bovine serum (FBS) supplemented group and an autologous serum (Auto Serum) supplemented group. Samples collected from 6 cancer patients were studied for the presence or absence of the CTCs-inducing ability.

### <Results>

The results are shown in Table 1. As shown in the table, as to the RPMI-1640 culture medium, no marked difference was seen in cancer cell yield efficiency between the autologous serum supplemented group and the FBS supplemented group, and the yield efficiency was low in both the groups.

**Table. 1**

| Patient | CTC number | |
|---|---|---|
| | Average ± SE | |
| | FBS | Auto Serum |
| G.N. | **2 ± 0** | **2 ± 0** |
| H.Y.1 | **4 ± 0** | **5 ± 1** |
| H.Y.2 | **0 ± 0** | **0 ± 0** |
| I.A. | **1.3 ± 0** | **2 ± 0** |
| K.M. | **0 ± 0** | **0 ± 0** |
| H.Y.3 | **0 ± 0** | **0 + 0** |

### (Test 2: Study on CTCs-inducing ability using RPMI-1640 and palmitic acid)

RPMI-1640 supplemented with palmitic acid was used as a culture medium, and this was used as a common culture medium. The culture medium was divided into a fetal bovine serum (FBS) supplemented group and an autologous serum (Auto Serum) supplemented group. Samples collected from 6 cancer patients were studied for the presence or absence of the CTCs-inducing ability.

### <Results>

The results are shown in Table 2. As shown in the table, as to the RPMI-1640 culture medium supplemented with palmitic acid, no marked difference was seen in cancer cell yield efficiency between the autologous serum supplemented group and the FBS supplemented group, except for (*), and the yield efficiency was low in both the groups.

**Table. 2**

| Patient | CTC number | |
|---|---|---|
| | Average ±SE | |
| | RPM11640+Palmitic Acid | |
| | FBS | Auto Serum |
| G. N. | **5.6 ± 1** | **4.3 ± 1** |
| H.Y.1 | **2.6 ± 0** | **2.6 ± 0** |
| H.Y.2 | **0.6 ± 0** | **0.6 ± 0** |
| I.A. | **1.6 + 0** | **137 ± 14(*)** |
| K.M. | **0 ± 0** | **5.3 ± 0** |
| H.Y.3 | **1 ± 0** | **1.6 ± 0** |

### (Test 3: Study on CTCs-inducing ability using RPMI-1640 and AIM-V)

The presence or absence of the CTCs-inducing ability was compared between a RPMI-1640 culture medium and an AIM-V culture medium. RPMI-1640 was divided into 2 groups: a fetal bovine serum (FBS) supplemented group and an autologous serum (Auto Serum) supplemented group. Samples collected from 6 cancer patients were studied for the presence or absence of the CTCs-inducing ability.

### <Results>

The results are shown in Table 3. As shown in the table, the AIM-V culture medium exhibited, singly without serum, high cancer cell yield efficiency, as compared with any of the culture media in which fetal bovine serum (FBS) or autologous serum (Auto Serum) was added to the RPMI-1640 culture medium.

**Table. 3**

| Patient | GTC number | | |
|---|---|---|---|
| | Average ±SE | | |
| | AIM-V alone | RPMI1 640 | |
| | | FBS | Auto Serum |
| G.N. | **179 ± 15** | **2 ± 0** | **2 ± 0** |
| H.Y.1 | **69 ± 4** | **4 ± 0** | **5 ± 1** |
| H.Y.2 | **6.3 ± 1** | **0 ± 0** | **0 ± 0** |
| I.A. | **49 ± 14** | **1.3 ± 0** | **2 ± 0** |
| K.M. | **28.6 ± 1** | **0 ± 0** | **0 ± 0** |
| H.Y.3 | **40 ± 4** | **0 ± 0** | **0 ± 0** |

### (Test 4: Study on CTCs-inducing ability, of culture medium in which FBS was added to AIM-V culture medium)

The presence or absence of the CTCs-inducing ability was studied on the condition that fetal bovine serum (FBS) was added to an AIM-V culture medium.

### <Results>

The results are shown in Table 4. As shown in the table, the group of the AIM-V culture medium supplemented with fetal bovine serum (FBS) exhibited high cancer cell yield efficiency, though large difference was observed in cancer cell yield efficiency among the patient sources.

**Table. 4**

| Patient | CTC number |
|---|---|
| | Average±SE |
| | AIMV+FBS |
| G.N. | **227 ± 56** |
| H.Y.1 | **13 ± 0** |
| H.Y.2 | **4.3 ± 1** |
| LA. | **9.3 ± 0** |
| K.M. | **6.3 ± 1** |
| H.Y.3 | **6 ± 0** |

### (Test 5. Study on CTCs-inducing ability, of culture medium in which palmitic acid was added to AIM-V culture medium supplemented with FBS)

A culture medium in which fetal bovine serum (FBS) was added to an AIM-V culture medium was used in common. A group of the culture medium supplemented with palmitic acid was created. The CTCs-inducing ability was compared between the group and a palmitic acid non-supplemented group.

### <Results>

The results are shown in Table 5. As shown in the table, difference in CTC yield efficiency was seen among the patients in the palmitic acid non-supplemented group, as in the case of test 4. By contrast, the addition of palmitic acid (palmitic acid supplemented group) was confirmed to improve and potentiate CTC yield efficiency even in the patient sources with poor CTC yield efficiency in the palmitic acid non-supplemented group.

**Table.5**

| Patient | CTC number | | |
|---|---|---|---|
| | Average±SE | | |
| | AIM-V+FBS | | AIM-V+FBS+Palmitic Acid |
| G.N. | **227 ± 56** | > | **115 ± 7** |
| H.Y.1 | **13 ± 0** | < | **86.6 ± 18** |
| H.Y.2 | **4.3 ± 1** | < | **99 ± 9** |
| I.A. | **9.3 ± 0** | < | **136.8 ± 21** |
| K.M. | **6.3 ± 1** | < | **74 ± 2** |
| H.Y.3 | **6 ± 0** | < | **200 ± 13** |

### (Test 6. Study on CTCs-inducing ability, of AIM-V culture medium alone and AIM-V culture medium supplemented with autologous serum (Auto Serum))

A group of an AIM-V culture medium alone and a group of AIM-V supplemented with patient's autologous serum (Auto Serum) were prepared. The CTCs-inducing ability was compared between both the culture media.

### <Results>

The results are shown in Table 6. As shown in the table, as to the group of AIM-V supplemented with patient's autologous serum (Auto Serum), CTC yield efficiency was improved in most of patient sources except for the case with (*). High CTC yield efficiency was observed in any of the cases.

**Table. 6**

| Patient | CTC number | | | |
|---|---|---|---|---|
| | Average±SE | | | |
| | AIM-V alone | | AIM-V+Auto Serum | |
| G.N. | **179.3 ± 15** | < | **246.3 ± 39** | |
| H.Y.1 | **69 ± 4** | << | **377 ± 55** | |
| H.Y.2 | **6.3 ± 1** | < | **54.6 ± 7** | |
| I.A. | **49 ± 14** | > | **27.6 ± 0** | **(*)** |
| K.M. | **28.6 ± 1** | < | **46 ± 4** | |
| H.Y.3 | **40 ± 4** | << | **480 ± 66** | |

### (Test 7. Study on CTCs-inducing ability, of culture medium in which palmitic acid was added to AIM-V culture medium supplemented with autologous serum (Auto Serum))

A culture medium in which patient's autologous serum (Auto Serum) was added to an AIM-V culture medium was used as a common culture medium. A group of the culture medium supplemented with palmitic acid was created. The CTCs-inducing ability was compared between the group and a palmitic acid non-supplemented group.

### <Results>

The results are shown in Table 7. As shown in the table, two contrasting trends were shown in CTC yield efficiency between the palmitic acid supplemented group and the palmitic acid non-supplemented group of the group of the culture medium in which patient's autologous serum (Auto Serum) was added to the AIM-V culture medium. Specifically, the addition of palmitic acid exhibited a decreasing trend in the patient sources having high CTC yield efficiency without the addition of palmitic acid. On the other hand, a rise in CTC yield efficiency was observed by the addition of palmitic acid in the patient sources having low CTC yield efficiency without the addition of palmitic acid.

**Table. 7**

| Patient | CTC number | |
|---|---|---|
| | Average ±SE | |
| | AIM-V+Auto Serum | AIM-V+Auto Serum+Palmitic Acid |
| G.N. | **243.6±39** | **118.6± 4** |
| H.Y.1 | **377 ±55** | **128.3± 14** |
| H.Y.2 | **54.6± 7** | **85 ± 2** |
| I.A. | **27.6± 0** | **497.3±12** |
| K.M. | **46 ± 4** | **117.5± 3** |
| H.Y.3 | **480 ±66** | **257 ± 21** |

### (Observation under fluorescence microscope)

Results (photograph) of observing, under a fluorescence microscope, one obtained in the CTC yield test using the AIM-V culture medium and the RPMI-1640 culture medium studied in tests 1 and 2 described above are shown in Figure 1. Figure (1-a) shows results of obtaining CTCs using the AIM-V culture medium, and Figure (1-b) shows results of obtaining CTCs in the case of using the RPMI-1640 culture medium. In the case of using the AIM-V culture medium, a large number of CTC cells were observed, and the CTCs-inducing ability was exhibited. In the case of using the RPMI-1640 culture medium, no CTC cells were observed.

### (Comprehensive evaluation)

From the test results mentioned above, the following conclusion was obtained.
(i) In both the groups of RPMI-1640 alone and RPMI-1640 + 5% Auto Serum (autologous serum) supplemented, CTCs were unable to be induced, separated, or obtained at a unit of 0.1% or more of the number of peripheral blood-derived mononuclear cells (PBMCs) seeded (1 × 10⁴ cells/well).
(ii) As to AIM-V alone, CTCs were able to be induced, separated, and obtained in the range of 0.1 to 0.2% of the seeded peripheral blood-derived mononuclear cells (PBMCs).
(iii) In the AIM-V + 5% Auto Serum (autologous serum) supplemented group, the rate of CTC obtainment was increased to 2.2 to 2.7%, which was 14 times to 20 times the case of AIM-V alone.
(iv) When RPMI-1640 was added at a ratio of 50% to AIM-V, the rate of CTC obtainment was reduced to the same % as that in the RPMI-1640 + 5% Auto Serum (autologous serum) supplemented group.
(v) The trend of (iv) described above was also seen in the addition of 25% RPMI-1640 and the addition of 12.5% RPMI-1640, and no rise in the CTC-obtaining effect was observed.

From this test result, it was confirmed that: AIM-V is useful as a culture medium used in the induction, separation, or obtainment of CTC; and the CTC-obtaining effect can be enhanced by adding autologous serum (AS) to the culture medium.

### Example 3

### [Selection test (II) of efficient culture medium for cancer cell (CTC) obtainment - influence of additive]

### <Culture medium to be tested>

The following culture media were prepared and used as culture media to be tested.
(1) AIM-V (serum-free culture medium)
(2) AS (Auto Serum: autologous serum)
(3) LPS (lipopolysaccharide)
(4) IL-1α (interleukin 1α)
(5) IL-1β (interleukin 1β)
(6) Palmitic Acid

### <Experimental method>

Prepared peripheral blood mononuclear cells (PBMCs) of a cancer patient were seeded to each well according to the experimental method described in Example 1 using the culture media described above, to test the influence of an additive in each culture medium on cancer cell (CTC) yield efficiency (cancer cell detection accuracy).

### (Test 8: Influence of LPS addition to AIM-V culture medium)

An AIM-V culture medium (AIM-V: Group A) was used as a basis. A group in which AS (autologous serum) was added thereto (AIM-V + AS: Group B), and further, groups in which LPS (lipopolysaccharide) was added at a basic concentration of 1 conc. (Groups E and F) or at each concentration of 0.1 conc. to 100 conc. (Groups GH and GD) to these groups were created. Then, a CTC-isolating effect was studied among these groups.

### <Results>

The results are shown in Table 8. As shown in the table, as to the groups of the AIM-V culture medium supplemented with LPS, a higher effect was seen in the group with a small amount of LPS added than in AIM-V alone, whereas CTC isolation efficiency was drastically decreased in the LPS supplemented AIM-V + AS group at any concentration of LPS.

**Table 8. Influence of LPS addition**

| Group | | Average±SE |
|---|---|---|
| | | |
| A | AIMV | 26.7±2 |
| B | AIMV+AS | 114 ±8 |
| | | |
| C | AIMV+LPS(x10 conc.) | 0 ±0 |
| D | AIMV+AS+LPS(x10 conc.) | 0 ±0 |
| | | |
| E | AIMV+LPS(x1conc.) | 3 ±1 |
| F | AIMV+AS+LPS(x1conc.) | 8.3±0 |
| | | |
| G | AIMV+LPS(x0.1conc.) | 45.7±8 |
| H | AIMV+AS+LPS(x0.1conc.) | 19 ±5 |

### (Test 9: Influence of IL-1α addition to AIM-V culture medium)

An AIM-V culture medium (AIM-V: Group A) was used as a basis. A group in which AS (autologous serum) was added thereto (AIM-V + AS: Group B), and further, groups in which IL-1α (interleukin 1α) was added at a basic concentration of 1 conc. (Groups E and F) or at each concentration of 0.1 conc. to 100 conc. (Groups GH and GD) to these groups were created. Then, a CTC-isolating effect was studied among these groups.

### <Results>

The results are shown in Table 9. As shown in the table, as to the groups of the AIM-V culture medium supplemented with IL-1α, only an effect equivalent to or lower than that of AIM-V alone was observed, irrespective of the larger or smaller amount of IL-1α added (Groups C, E, and G). CTC isolation efficiency was decreased in the IL-1α supplemented AIM-V + AS group at any concentration of IL-1α, compared with the non-supplemented groups.

**Table 9. Influence of IL-1α addition**

| | | Average±SE |
|---|---|---|
| | | |
| A | AIMV | 19.3±0 |
| B | AIMV+AS | 32 ±5 |
| | | |
| C | AIMV+IL-1*α*(x10 conc.) | 20 ±2 |
| D | AIMV+AS+IL-1 *α*(x10 conc.) | 30 ±4 |
| | | |
| E | AIMV+IL-1 *α*(x1conc.) | 6.3±0 |
| F | AIMV+AS+IL-1*α*(x1conc.) | 12.7±0 |
| | | |
| G | AIMV+IL-1*α*(x0.1conc.) | 15.3±3 |
| H | AIMV+AS+IL-1*α*(x0.1conc.) | 24.7±6 |

### (Test 10: Influence of IL-1β addition to AIM-V culture medium)

An AIM-V culture medium (AIM-V: Group A) was used as a basis. A group in which AS (autologous serum) was added thereto (AIM-V + AS: Group B), and further, groups in which IL-1β (interleukin 1β) was added at a basic concentration of 1 conc. (Groups E and F) or at each concentration of 0.1 conc. to 100 conc. (Groups GH and GD) to these groups were created. Then, a CTC-isolating effect was studied among these groups.

### <Results>

The results are shown in Table 10. As shown in the table, as to the groups of the AIM-V culture medium supplemented with IL-1β, only an effect equivalent to or slightly higher than that of AIM-V alone was observed, according to the amount of IL-1β added (Groups C, E, and G). Only CTC yield efficiency equivalent to that of the non-supplemented groups was observed in the IL-1β supplemented AIM-V + AS group at any concentration of IL-1β.

**Table 10. Influence of IL-1β addition**

| | | Average±SE |
|---|---|---|
| | | |
| A | AIMV | 31±1 |
| B | AIMV+AS | 41±2 |
| | | |
| C | AIMV+IL-1*β*(x10 conc.) | 41±2 |
| D | AIMV+AS+IL-1*β*(x10 conc.) | 37±2 |
| | | |
| E | AIMV+IL-1*β*(x1conc.) | 35±1 |
| F | AIMV+AS+IL-1*β*(x1conc.) | 40±2 |
| | | |
| G | AIMV+IL-1*β*(x0.1conc.) | 27±2 |
| H | AIMV+AS+IL-1*β*(x0.1conc.) | 31±9 |

### (Test 11: Influence of palmitic acid addition to AIM-V culture medium)

An AIM-V culture medium (AIM-V: Group A) was used as a basis. A group in which AS (autologous serum) was added thereto (AIM-V + AS: Group B), and further, groups in which palmitic acid was added at a basic concentration of 1 conc. (Groups G and H) or at each concentration of 0.25 conc. to 4 conc. (Groups KL, IJ, GH, EF, and CD) to these groups were created. Then, a CTC-isolating effect was studied among these groups.

### <Results>

The results are shown in Table 11. As shown in the table, as to the groups of the AIM-V culture medium supplemented with palmitic acid, 2- to 3-fold rise in CTC yield efficiency as compared with the non-supplemented groups was confirmed, almost irrespective of the amount of palmitic acid added (Groups C, E, G, I, and K). No concentration dependency was seen, and only CTC yield efficiency equivalent to or lower than that in the non-supplemented groups was observed, in the palmitic acid supplemented AIM-V + AS group at any concentration of palmitic acid.

**Table 11. Influence of palmitic acid addition**

| | | Average±SE |
|---|---|---|
| | | |
| A | AIMV | 45.3±3 |
| B | AIMV+AS | 192±12 |
| | | |
| C | AIMV+Palmitic Acid (x4 conc.) | 95.3±2 |
| D | AIMV+AS+Palmitic Acid (x4 conc.) | 123.3±8 |
| | | |
| E | AIMV+Palmitic Acid (x2conc.) | 142.7±19 |
| F | AIMV+AS+Palmitic Acid (x2conc.) | 182.7±15 |
| | | |
| G | AIMV+Palmitic Acid (x1 conc.) | 99.7±3 |
| H | AIMV+AS+Palmitic Acid (x1 conc.) | 184.7±30 |
| | | |
| I | AIMV+Palmitic Acid (x0.5conc.) | 112.3±21 |
| J | AIMV+AS+Palmitic Acid (x0.5conc.) | 194.7±20 |
| | | |
| K | AIMV+Palmitic Acid (x0.25conc.) | 97.3±11 |
| L | AIMV+AS+Palmitic Acid (x0.25conc.) | 176.7±37 |

### (Comprehensive evaluation)

From the test results mentioned above, the following conclusion was obtained: as a result of studying enhancement in CTC-obtaining effect by adding an additive component to an AIM-V culture medium, studying various candidates of additive components (LPS, ConA, PHA, IL-1α, and IL-1β) including palmitic acid as novel CTC-separating/obtaining factors, and carrying out search for the safest and most efficient factor, it was confirmed that the addition of palmitic acid to the AIM-V culture medium produces the best consequence for the stable isolation of CTCs. Although a CTC-inducing effect was also slightly observed in LPS during the course of this study, LPS is a substance having very strong toxicity and was therefore judged as being inappropriate as a reagent for safety reasons in light of various purposes of research and clinical application technique development after CTC separation and excluded from subsequent experiments.

### Example 4

### [Confirmation of morphological change in cancer cells (CTCs) obtained in culture medium for CTC proliferation]

Using the test method of Example 1, the morphological change in the cancer cells (CTCs) obtained using the culture medium for CTC proliferation in Example 2 was examined based on microscopic images. The microscopic photographs are shown in Figures 2 to 7. In each figures, (figure-a) and (figure-b) show results of studying CTCs from different patients (microscopic photographs of cancer cells (CTCs) from different patients) for the morphological features of CTCs under the culture conditions of a CTC cell proliferation experiment, i.e., the culture conditions such as a culture medium for proliferation and the presence or absence and concentration of serum or a factor such as palmitic acid. The experiment was aimed at studying the presence or absence of expression of morphological difference in CTC cells depending on difference among proliferation conditions, and (a) and (b) were intended to verify that even CTCs derived from different patients exhibit the same cell morphology under the same culture condition.

The concentration of palmitic acid in the concentration of the culture medium to be tested according to Table 12 below.

**[Table 12]**

| Palmitic Acid | *µ*g/ml (r) |
|---|---|
| ×0.25 | 6.41 |
| ×0.5 | 12.821 |
| ×1 | 25.642 |
| ×2 | 51.284 |
| ×4 | 102.57 |

### <1. Observation of morphological change in CTCs obtained by culture using AIM-culture medium alone>

A fluorescence microscopic image of CTCs obtained by culture using an AIM-V culture medium alone is shown in Figure 2 (2-a; 2-b). In the cell morphology of CTCs obtained by culture using the AIM-V culture medium alone, an abnormal size or a strong stained portion was not particularly seen in the nuclei or granular components in the cytoplasms.

### <2. Observation of morphological change in CTCs obtained by culture using AIM-V culture medium supplemented with palmitic acid (× 1 conc.)>

A fluorescence microscopic image of CTCs obtained by culture using an AIM-V culture medium supplemented with palmitic acid (+ palmitic acid (× 1 concentration)) is shown in Figure 3 (3-a; 3-b). In the cell morphology of CTCs obtained by culture using the AIM-V culture medium supplemented with palmitic acid (+ palmitic acid (× 1 concentration)), expansion in size or a strong stained portion was not particularly seen in the nuclei or granular components in the cytoplasms, though slight increase in nuclear components was seen, as compared with the case of the palmitic acid non-supplemented group.

### <3. Observation of morphological change in CTCs obtained by culture using AIM-V culture medium supplemented with palmitic acid (× 4 conc.)>

A fluorescence microscopic image of CTCs obtained by culture using an AIM-V culture medium supplemented with palmitic acid (+ palmitic acid (× 4 concentration)) is shown in Figure 4 (4-a; 4-b). In the cell morphology of CTCs obtained by culture using the AIM-V culture medium supplemented with palmitic acid (+ palmitic acid (× 4 concentration)), trends of expansion in size and fusion of the cells were seen (Figure 4-b), and marked increase or strong staining in nuclear components or the nuclei or granular components in the cytoplasms was observed in the nuclei or granular components in the cytoplasms, as compared with the case of the palmitic acid non-supplemented group (Figure 2-a; 2-b) or the (× 1 concentration) supplemented group (Figure 3-a; 3-b).

### <4. Observation of morphological change in CTCs obtained by culture using AIM-V + 5% autologous serum culture medium>

A fluorescence microscopic image of CTCs obtained by culture using an AIM-V + 5% autologous serum culture medium is shown in Figure 5 (5-a; 5-b). In the cell morphology of CTCs obtained by culture using the AIM-V + 5% autologous serum culture medium, an abnormal size or a strong stained portion was not particularly seen in the cells or the nuclei or granular components in the cytoplasms.

### <5. Observation of morphological change in CTCs obtained by culture using culture medium under condition of AIM-V + 5% autologous serum + palmitic acid (× 1 conc.)>

A fluorescence microscopic image of CTCs obtained by culture using an AIM-V + 5% autologous serum + palmitic acid (× 1 concentration) culture medium is shown in Figure 6 (6-a; 6-b). In the cell morphology of CTCs obtained by culture using the AIM-V + 5% autologous serum + palmitic acid (× 1 concentration) culture medium, an abnormal size or a strong stained portion was not particularly seen in the cells or the nuclei or granular components in the cytoplasms.

### <6. Observation of morphological change in CTCs obtained by culture using culture medium under condition of AIM-V + 5% autologous serum + palmitic acid (× 4 conc.)>

A fluorescence microscopic image of CTCs obtained by culture using an AIM-V + 5% autologous serum + palmitic acid (× 4 concentration) culture medium is shown in Figure 7 (7-a; 7-b). In the cell morphology of CTCs obtained by culture using the AIM-V + 5% autologous serum + palmitic acid (× 4 concentration) culture medium, trends of expansion in size and fusion of the cells were seen (Figure 7-a), and marked increase or strong staining in nuclear components or granular components in the cytoplasms was observed in the nuclei or granular components in the cytoplasms, as compared with the palmitic acid non-supplemented group (Figure 5-a; 5-b) or the × 1 concentration supplemented group (Figure 6-a; 6-b). This morphological change is considered to increase abnormal proliferation of the nuclei, subcellular organelles, mitochondria which play a leading role in energy production, etc. through the proliferation or activation of CTC cells under a condition of a palmitic acid (× 4 concentration) culture medium. As a result, it is considered that in microscopic images, nucleic acids or intracellular granules are increased, and the nuclei or the cytoplasms further interfere with light permeability so that a strong staining is observed.

### (Comprehensive evaluation)

From the results of testing morphological change in CTCs (CTSCs) mentioned above, the following conclusion was obtained: it was confirmed that: CTCs can be reliably and effectively obtained by proliferating and obtaining CTCs using an AIM-V culture medium, or growth medium for a CTC in which an additive component such as autologous serum and/or palmitic acid has been added to the culture medium; and in this respect, it is possible to proliferate and obtain CTCs without causing morphological change in cell morphology. Specifically, in the case of proliferating and obtaining CTCs (CTSCs), it is important for confirming the properties or characteristics of CTCs that artificial change is not induced in the morphology of obtained cells. Thus, a method that can stably proliferate and obtain CTCs under a condition that induces no particular change in the morphology or character of CTCs is demanded. From the results of experiments described above, it was confirmed that the method can meet the requirements, and reliably proliferate and obtain CTCs (CTSCs).

### Example 5

### [Confirmation and identification using cell membrane antigen of obtained CTCs]

As shown in Examples below, CTCs separated and obtained from the peripheral blood of each patient were confirmed and identified by the fluorescent antibody staining method using each of antigens CD44, CD45, CD47, CKII, and EpCAM on membrane surface. In a photograph of each figure about the results of each test, each (figure-a) shows a microscopic photographic image of CTCs, and each (figure-b) shows a fluorescence microscopic photographic image of staining using the membrane surface antigen.

### <Antigen staining method>

In the antigen staining method, samples were prepared, stained, microscopically examined, observed, and photographed according to the following procedures using a FITIC (fluorescein isothiocyanate) label.

### (I. Preparation of various peripheral blood-separated cells and antigen staining with FITC-labeled primary antibody)

(1) Enzymatic treatment of attached cells (starting at (4) for non-attached cells).
(2) Trypsin EDTA treatment.
(3) Addition of a RPMI medium supplemented with 5% FBS to a single cell group, termination of enzymatic reaction, and aspiration.
(4) Centrifugation (1200 to 1500 rpm, 4°C, 7 min).
(5) Washing and vortex with (-)PBS under cold storage at 4°C, and centrifugation × 2.
(6) Cell number counting and dispensing: 1 × 10⁶ cells/100 µl/tube.
(7) Aluminum foil is wrapped around each tube of the cells for light shielding, and the tube is placed for 30 minutes under cold storage at 4°C to reduce the metabolic activity of the cells (inhibition of mobility or movement of the surface antigen to the inside or outside of the cell membrane).
(8) Addition of the antibody: 1 µg/2 µl/1 × 10⁶ spin down cells (medium-removed cell group), and staining for 1 hour under cold storage at 4°C and under light shielding.
(9) Washing and vortex with (-)PBS under cold storage at 4°C, and centrifugation × 2: centrifugal separation (1200 to 1500 rpm, 4°C, 7 min).
(10) An appropriate amount of (-)PBS is added to the cells, which are then added to a glass slide or a plate and microscopically examined.
(11) Observation and photographing under a fluorescence microscope.

### (II. Preparation of various peripheral blood-separated cells and staining with primary antibody against each antigen and then the antigen staining with FITC-labeled secondary antibody)

(1) Enzymatic treatment of attached cells (starting at (4) for non-attached cells).
(2) Trypsin EDTA treatment.
(3) Addition of a RPMI medium supplemented with 5% FBS to a single cell group, termination of enzymatic reaction, and aspiration.
(4) Centrifugation (1200 to 1500 rpm, 4°C, 7 min).
(5) Washing and vortex with (-)PBS under cold storage at 4°C, and centrifugation × 2.
(6) Cell number counting and dispensing: 1 × 10⁶ cells/100 µl/tube.
(7) Aluminum foil is wrapped around each tube of the cells for light shielding, and the tube is placed for 30 minutes under cold storage at 4°C to reduce the metabolic activity of the cells (inhibition of mobility or movement of the surface antigen to the inside or outside of the cell membrane).
(8) Addition of the primary antibody: 1 µg/2 µl/1 × 10⁶ spin down cells (medium-removed cell group), and staining for 1 hour under cold storage at 4°C and under light shielding.
(9) Washing and vortex with (-)PBS under cold storage at 4°C, and centrifugation × 2: centrifugal separation (1200 to 1500 rpm, 4°C, 7 min).
(10) Addition of the secondary antibody: 1 µg/2 µl/1 × 10⁶ spin down cells (medium-removed cell group), and staining for 1 hour under cold storage at 4°C and under light shielding.
(11) Washing and vortex with (-)PBS under cold storage at 4°C, and centrifugation × 2: centrifugal separation (1200 to 1500 rpm, 4°C, 7 min).
(12) An appropriate amount of (-)PBS is added to the cells, which are then added to a glass slide or a plate and microscopically examined.
(13) Observation and photographing under a fluorescence microscope.

### Example 6

### [Confirmation and identification of CTCs using membrane antigen CD44]

CTCs obtained from the peripheral blood of each patient by the method of Example 1 were confirmed and identified by using the membrane antigen CD44 for the CTCs.

### <Confirmation and identification (I) of CTCs using membrane antigen CD44>

Microscopic photographic images of CTCs obtained from the peripheral blood of 3 patients: (1) K.H. (Gastric Ca.: gastric cancer, liver meta.: liver metastasis), (2) T.K. (Tongue Ca.: tongue cancer), and (3) K.H. (Gastric Ca.: gastric cancer, liver meta.: liver metastasis), and fluorescent antibody microscope photographs thereof using the membrane antigen CD44 are shown in Figures 8 to 10. (Figures 8-a to 10-a) show the microscopic photographic images, and (Figures 8-b to 10-b) show the fluorescent antibody microscope photographs. As shown in the photographs, the CTCs obtained from the patients (1) to (3) described above did not exhibit staining with CD44.

### <Confirmation and identification (II) of CTCs using membrane antigen CD44>

Microscopic photographic images of CTCs obtained from the peripheral blood of 3 patients: (1) T.K. (Tongue Ca.: tongue cancer), (2) S.K. (Kerato-cystic Ca.: keratocystic cancer), and (3) S.O. (Lung Ca.: lung cancer), and fluorescent antibody microscope photographs thereof using the membrane antigen CD44 are shown in Figures 11 to 13. (Figures 12-a to 13-a) show the microscopic photographic images, and (Figures 12-b to 13-b) show the fluorescent antibody microscope photographs. As shown in the photographs (Figures 12 and 13), the CTCs obtained from the patients (2) and (3) described above exhibited staining with CD44, and the microscopic photographic image and the fluorescent antibody microscope photograph were consistent with each other, confirming that the obtained CTCs were CTCs of the cancers described above.

### <Confirmation and identification (III) of CTCs using membrane antigen CD44>

Microscopic photographic images of CTCs obtained from the peripheral blood of 2 patients: (1) H.Y. (Breast Ca.: breast cancer) and (2) Y.H. (Lung Ca.: lung cancer), and fluorescent antibody microscope photographs thereof using the membrane antigen CD44 are shown in Figures 14 and 15. (Figures 14-a and 15-a) show the microscopic photographic images, and (Figures 14-b and 15-b) show the fluorescent antibody microscope photographs. As shown in the photographs (Figures 14 and 15), the CTCs obtained from the patients (1) and (2) described above exhibited staining with CD44, and the microscopic photographic image and the fluorescent antibody microscope photograph were consistent with each other, confirming that the obtained CTCs were CTCs of the cancers described above.

### Example 7

### [Confirmation and identification of CTCs using membrane antigen CD45]

CTCs obtained from the peripheral blood of each patient by the method of Example 1 were confirmed and identified by using the membrane antigen CD45 for the CTCs.

Microscopic photographic images of CTCs obtained from the peripheral blood of 2 patients: (1) H.Y. (Breast Ca.: breast cancer) and (2) Y.H. (Lung Ca.: lung cancer), and fluorescent antibody microscope photographs thereof using the membrane antigen CD45 are shown in Figures 16 to 18. (Figures 16-a to 18-a) show the microscopic photographic images, and (Figures 16-b to 18-b) show the fluorescent antibody microscope photographs. As shown in the photographs (Figures 16 to 18), the CTCs obtained from the patients (1) and (2) described above exhibited staining with CD45, and the microscopic photographic image and the fluorescent antibody microscope photograph were consistent with each other, confirming that the obtained CTCs were CTCs of the cancers described above. In the microscopic photographs of the CTCs (CTSCs), the presence of the CTSC cells is indicated by arrow. In each photograph group (photographs 1 to 3: Figures 16 to 18), it is shown that a great majority of cells among the CTCs (CTSCs) seen in the left microscopic photographs are CD45 positive CTSC cells, as seen in the right fluorescence microscopic images. As indicated by double-sided arrow, it is evident that these CD45 positive CTSC cells have diversity in morphology and size and are not cells that can be discriminated by one index.

### Example 8

### [Confirmation and identification of CTCs using membrane antigen CD47]

CTCs obtained from the peripheral blood of each patient by the method of Example 1 were confirmed and identified by using the membrane antigen CD47 for the CTCs.

Microscopic photographic images of CTCs obtained from the peripheral blood of 5 patients: (1) G.N. (Breast Ca.: breast cancer, multiple meta.: multiple metastasis), (2) S.K. (Kerato-cystic Ca.: keratocystic cancer), (3) S.O. (Lung Ca.: lung cancer), (4) H.Y. (Breast Ca.: breast cancer), and (5) Y.H. (Lung Ca.: lung cancer), and fluorescent antibody microscope photographs thereof using the membrane antigen CD47 are shown in Figures 19 to 23. (Figures 19-a to 23-a) show the microscopic photographic images, and (Figures 19-b to 23-b) show the fluorescent antibody microscope photographs. As shown in the photographs (Figures 19 to 23), the CTCs obtained from the patients (1) to (5) described above exhibited staining with CD47, and the microscopic photographic image and the fluorescent antibody microscope photograph were consistent with each other, confirming that the obtained CTCs were CTCs of the cancers described above. In the photograph of each figure, the arrows indicate CD47 positive CTSCs. The CTSCs indicated by the white arrows are CD47 positive CTSCs among cancer cells proliferated and cultured as CTCs (CTSCs), showing that the cells are observed regardless of magnitude of size or morphology. Specifically, it was confirmed that the CD47 positive CTSCs are not cells having a certain morphology or size and have variations.

### Example 9

### [Confirmation and identification of CTCs using membrane antigen CKII]

CTCs obtained from the peripheral blood of each patient by the method of Example 1 were confirmed and identified by using the membrane antigen CKII for the CTCs.

Microscopic photographic images of CTCs obtained from the peripheral blood of 2 patients: (1) H.Y. (Breast Ca.: breast cancer) and (2) Y.H. (Lung Ca.: lung cancer), and fluorescent antibody microscope photographs thereof using the membrane antigen CKII are shown in Figures 24 and 25. (Figures 24-a and 25-a) show the microscopic photographic images, and (Figures 24-b and 25-b) show the fluorescent antibody microscope photographs. As shown in the photographs (Figures 24 and 25), the CTCs obtained from the patients (1) and (2) described above exhibited staining with CKII, and the microscopic photographic image and the fluorescent antibody microscope photograph were consistent with each other, confirming that the obtained CTCs were CTCs of the cancers described above.

### Example 10

### [Confirmation and identification of CTCs using membrane antigen EpCAM]

CTCs obtained from the peripheral blood of each patient by the method of Example 1 were confirmed and identified by using the membrane antigen EpCAM for the CTCs.

A microscopic photographic image of CTCs obtained from the peripheral blood of a patient: (1) H.Y. (Breast Ca.: breast cancer), and a fluorescent antibody microscope photograph thereof using the membrane antigen EpCAM are shown in Figure 26. (Figure 26-a) shows the microscopic photographic image, and (Figure 26-b) shows the fluorescent antibody microscope photograph. As shown in the photographs (Figure 26), only a very small number of cells expressing EpCAM on their cell membranes were observed in the CTCs obtained from the patient described above.

### Example 11

### [Study on tumorigenicity or long-term survival of CTCs in nude mice]

In order to study whether or not CTCs obtained from the peripheral blood of a patient by the method of Example 1 would retain CTC cell functions, the tumorigenicity or long-term survival of the obtained CTCs was confirmed.

### <Test method>

CTCs separated from a sample from patient H.Y. (Breast Ca.: breast cancer): CTC-HY-1 (HY-1) and CTCs separated from patient K.H. (Gastric Ca.: gastric cancer, liver meta.: liver metastasis): CTC-KH-1 (KH-1) were divided into two experimental groups "experimental group A" and "experimental group B". The CTCs proliferated, separated, and collected from the patients were transplanted as given below.
(Experimental group A): For two nude mice (Nude 1-1; Nude 1-2), 1 × 10⁶ CTCs (HY-1) for "Nude 1-1" and CTCs (KH-1) for "Nude 1-2" were subcutaneously transplanted to the backs of the nude mice, and the presence or absence of tumorigenicity was studied for an observation period of 3 months in total. After the period, peripheral blood and the spleen were collected from the nude mice, and the separation and culture of CTCs were carried out. By the fluorescent antibody method using a non-fluorescent emission image (Control) and a membrane surface CD45 antigen, proliferated cells remaining in the CTC transplantation groups was stained, and the surviving cells were confirmed to be the transplanted CTCs
(Experimental group B): For two nude mice (Nude 2-1; Nude 2-2), 1 × 10⁶ CTCs (HY-1) for "Nude 2-1" and CTCs (KH-1) for "Nude 2-2" were subcutaneously transplanted to the backs of the nude mice, and the same amount as above of the cells was also intraperitoneally transplanted. As in the case of experimental group A, proliferated cells remaining in the CTC transplantation groups was stained, and the surviving cells were confirmed to be the transplanted CTCs.

### <Results>

Microscopic photographic images (non-fluorescent emission images) of cells remaining in the CTC transplantation groups and fluorescent antibody microscope photographs (fluorescent emission images) thereof using the membrane antigen CD45 are shown in Figures 27 to 30, In the figures, (27-a) and (27-b) show the microscopic photograph (Control) and the fluorescent antibody microscope photograph (fluorescent emission image), respectively, of the cells remaining in the CTC transplantation group in the case where the CTCs (HY-1) were transplanted in "Nude 1-1", and (28-a) and (28-b) show the microscopic photograph (Control) and the fluorescent antibody microscope photograph (fluorescent emission image), respectively, of the cells remaining in the CTC transplantation group in the case where the CTCs (KH-1) were transplanted in "Nude 1-2". In the figures, (29-a) and (29-b) show the microscopic photograph (Control) and the fluorescent antibody microscope photograph (fluorescent emission image), respectively, of the cells remaining in the CTC transplantation group in the case where the CTCs (HY-1) were transplanted in "Nude 2-1", and (30-a) and (30-b) show the microscopic photograph (Control) and the fluorescent antibody microscope photograph (fluorescent emission image), respectively, of the cells remaining in the CTC transplantation group in the case where the CTCs (KH-1) were transplanted in "Nude 2-2". As shown in the photographs (Figures 27 to 30), the cells remaining in the CTC transplantation groups exhibited staining with the membrane antigen CD45, which was consistent with the microscopic photographic image, confirming that the cells remaining in the CTC transplantation groups were CTCs of the cancers described above. A microscopic photographic image (31-a) of normal small intestine tissue cells and a fluorescence microscopic photographic image (31-b) of the cells by CD45 staining are shown as a reference. As seen in Figure 31, the normal small intestine tissue cells did not exhibit staining with CD45.

### Example 12

### [Detection (I) of CD47 positive cells (CTSCs) using established cancer cell line (UTC-8)]

An existing established cancer cell line (UTC-8: International Patent Organism Depositary, National Institute of Technology and Evaluation, Deposition No: FERM BP-08611) was used to test the detection of CD47 positive cells (CTSCs) for the established cancer cell line.

### <Test method>

CTC cells were proliferated and obtained by the method of Example 1 using the established cancer cell line (UTC-8) as a sample, and then, CD47 positive cells were detected using the cell membrane antigen CD47. The results were displayed by a microscopic image of non-fluorochrome staining and a CD47 positive cell image by the fluorochrome staining method.

The results are shown in Figure 32. In Figure 32, (32-a) shows the microscopic image of non-fluorochrome staining, and (32-b) shows the CD47 positive cell image by the fluorochrome staining method. In the figures, the thick arrows (=>) indicate CD47 positive CTSCs, and the double-sided arrows (↔) indicate CTC cancer cells differing in both the size and morphology of CD47 negative cells. As a result, a very small number of CD47 positive CTSCs were observed in a great majority of cancer cells passaged over longer than 20 years, as seen in this microscopic field (× 200). It was also shown that most of cancer cells are cells morphologically having a round or spindle shape or a shape similar thereto, or an irregular form that exhibits neither top-bottom nor bilateral symmetry. On the other hand, it was revealed that the shape of the CD47 positive CTSCs is a unique shape with dendritic cell-like irregular protrusions.

### Example 13

### [Detection (II) of CD47 positive cells (CTSCs) using established cancer cell line (UTC-8)]

The existing established cancer cell line (UTC-8: FERM BP-08611) was used to test again the detection of CD47 positive cells (CTSCs) for the established cancer cell line, as in the case of Example 12.

### <Test method>

CTC cells were proliferated and obtained by the method of Example 1 using the established cancer cell line (UTC-8) as a sample, and then, CD47 positive cells were detected using the cell membrane antigen CD47 , as in the case of Example 12. The results were displayed by a microscopic image of non-fluorochrome staining and a CD47 positive cell image by the fluorochrome staining method.

The results are shown in Figure 33. In Figure 33, (33-a) shows the microscopic image of non-fluorochrome staining, and (33-b) shows the CD47 positive cell image by the fluorochrome staining method. In the figures, the thick arrows (=>) indicate CD47 positive CTSCs, and the double-sided arrows (↔) indicate CTC cancer cells differing in both the size and morphology of CD47 negative cells. As a result, an outcome similar to that in the case of Example 12 was obtained. Specifically, a very small number of CD47 positive CTSCs were observed in the established cancer cell line (UTC-8), as seen in this microscopic field (× 200). It was also shown that most of cancer cells are cells morphologically having a round or spindle shape or a shape similar thereto, or an irregular form that exhibits neither top-bottom nor bilateral symmetry. On the other hand, it was revealed that the shape of the CD47 positive CTSCs is a unique shape with dendritic cell-like irregular protrusions. The morphological features were clearly confirmed.

### Industrial Applicability

The present invention provides a method for detecting or separating/obtaining CTC, which is capable of reliably and stably detecting or separating/obtaining a circulating tumor cell and a circulating tumor stem cell present in trace amounts in a biological circulating body fluid such as blood or lymph, even in the state where the cancer type of the tumor cells cannot be determined yet and the state where the tumor cells are present in trace amounts in the biological circulating body fluid. The method for detecting or separating/obtaining CTC according to the present invention enables not only CTCs but CTSCs to be detected or separated and provides means useful for the basic elucidation of cancer or a clinical approach therefor.

## Claims

1. A method for detecting or separating/obtaining a circulating tumor cell and/or a circulating tumor stem cell in a biological circulating body fluid, comprising the following treatment steps (1) to (4):
(1) a first step of pretreating a sample from the biological circulating body fluid to obtain a mononuclear cell phase;
(2) a second step of providing a well plate in which a culture medium consisting of a serum-free cell growth medium for circulating tumor cell and/or circulating tumor stem cell has been injected, and seeding thereto the mononuclear cell obtained in the first step, followed by incubation;
(3) a third step of removing the culture medium from a well of the plate obtained by the incubation in the second step; and
(4) a fourth step of detecting or separating/obtaining an adherent tumor cell attached to the well of the plateafter the third step.

2. The method for detecting or separating/obtaining a circulating tumor cell and/or a circulating tumor stem cell in a biological circulating body fluid according to claim 1, wherein in the second step, the culture medium consisting of a serum-free cell growth medium for circulating tumor cell and/or circulating tumor stem cell, for seeding and incubating the mononuclear cell obtained in the first step is an AIM-V-based culture medium.

3. The method for detecting or separating/obtaining a circulating tumor cell and/or a circulating tumor stem cell in a biological circulating body fluid according to claim 1 or 2, wherein in the second step, the AIM-V-based culture medium for seeding and incubating the mononuclear cell obtained in the first step is an AIM-V culture medium, or a culture mediumto which one or more components selected from autologous serum of a subject, healthy individual-derived AB serum, and palmitic acid or a salt thereof have been added to the AIM-V culture medium.

4. The method for detecting or separating/obtaining a circulating tumor cell and/or a circulating tumor stem cell in a biological circulating body fluid according to any one of claims 1 to 3, wherein the sample from the biological circulating body fluid is a blood sample from peripheral blood.

5. The method for detecting or separating/obtaining a circulating tumor cell and/or a circulating tumor stem cell in a biological circulating body fluid according to any one of claims 1 to 3, wherein the pretreatment of the sample from the biological circulating body fluid in the first step is removal of a liquid component and a non-cellular component contained in the biological circulating body fluid.

6. The method for detecting or separating/obtaining a circulating tumor cell and/or a circulating tumor stem cell in a biological circulating body fluid according to any one of claims 1 to 5, wherein the incubation in the second step is performed with 37°C and 3 to 7 days as standard conditions of a proliferation temperature and a proliferation period.

7. The method for detecting or separating/obtaining a circulating tumor cell and/or a circulating tumor stem cell in a biological circulating body fluid according to claim 6, wherein the incubation in the second step is performed in an incubator adjusted to 5% CO₂.
